# EUROPEAN PATENT SPECIFICATION

(11) **EP 1 073 623 B1**
(45) Date of publication and mention of the grant of the patent: **11.04.2007**
(21) Application number: 99918686.9
(22) Date of filing: 19.04.1999
(51) Int. Cl.: C07C 229/00, C07C 233/01, C07C 237/06, A61K 31/19, A61K 31/195

(54) **SUBSTITUTED AMINO ACIDS AS ERYTHROPOIETIN MIMETICS**
SUBSTITUTIERTE AMINOSAEUREN ALS ERYTHROPOIETIN-MIMETICA
ACIDES AMINES SUBSTITUES EN TANT QU'IMITATIONS D'ERYTHROPOIETINE

(30) Priority: 20.04.1998 US 82392 P
(43) Date of publication of application: 07.02.2001
(73) Proprietor: Ortho-McNeil Pharmaceutical, Inc., Raritan, NJ 08869-0602 (US)
(72) Inventor: CONNOLLY, Peter, New Providence, NJ 07974 (US); MURRAY, William, Belle Mead, NJ 08502 (US)
(74) Representative: Mercer, Christopher Paul
(86) International application number: PCT/US1999/008582
(87) International publication number: WO 1999/054279

(56) References cited:
- WO-A-97/41526
- WRIGHTON N C ET AL: "SMALL PEPTIDES AS POTENT MIMETICS OF THE PROTEIN HORMONE ERYTHROPOIETIN" SCIENCE, AMERICAN ASSOCIATION FOR THE ADVANCEMENT OF SCIENCE,, US, vol. 273, 26 July 1996 (1996-07-26), pages 458-463, XP002021036 ISSN: 0036-8075
- JOHNSON D ET AL: "Identification of a 13 amino acid peptide mimetic of erythropoietin and description of amino acids critical for the mimetic activity of EMP1" BIOCHEMISTRY, AMERICAN CHEMICAL SOCIETY. EASTON, PA, US, vol. 37, no. 11, 17 March 1998 (1998-03-17), pages 3699-3710, XP002147315 ISSN: 0006-2960
- LI et al., "Activation of Cell Growth by Binding of Friend Spleen Focus-Forming Virus gp55 Glycoprotein to the Erythropoietin Receptor", NATURE, 22 February 1990, Vol. 343, No. 6260, pages 762-764, XP002921762
- KAWASE M., "Unusual Reactions of Secondary Amino Acids with Trifluoroacetic Anhydride a Novel Access to .alpha.-Trifluormethylated Acyloins", TETRAHEDRON LETTERS, January 1994, Vol. 35, No. 1, pages 149-152, XP002921763
- KUBOTA et al., "Wortmannin, a Specific Inhibitor of Phosphatidylinositol-3-Kinase, Inhibits Erythropoietin-Induced Erythroid Differentiation of K562 Cells", LEUKEMIA, April 1996, Vol. 10, No. 4, pages 720-726, XP002921764
- HOFFMAN et al., "An Improved Enantiospecific Synthesis of Statine and Statine Analogs via 4-(N,N-Dibenzylamino) -3-Keto Esters", THE JOURNAL OF ORGANIC CHEMISTRY, April 1997, Vol. 62, No. 7, pages 2292-2297, XP002921765
- BONJOCH et al., "Synthesis of Enantiopure(2R,3aS,7aS)-2-Ethyloctahydroind ol-6-one and its Fischer Indolization", TETRAHEDRON: ASYMMETRY, September 1997, Vol. 8, No. 18, pages 3143-3151, XP004090524

## Description

This invention relates to a series of small molecules which bind to the erythropoietin receptor and compete with the natural ligand for binding to said receptor. The invention includes pharmaceutical compositions containing these mimetics, their methods of production as well as medical uses of these mimetics.

Erythropoietin (EPO) is a glycoprotein hormone which is produced in the mammalian kidney and has a molecular weight of about 34,000 daltons. Its primary role is stimulation of mitotic cell division and differentiation of erythrocyte precursor cells. As a result this hormone regulates the production of erythrocytes, the hemoglobin contained therein and the blood's ability to carry oxygen. The commercial product Epogen ® is used in the treatment of anemia. This drug is produced by recombinant techniques and formulated in aqueous isotonic sodium chloride/sodium citrate. Even though it has been used successfully in the treatment of anemia, it is a costly drug that is administered intravenously. This method of administration is both costly and inconvenient for the patient; therefore it would be desirable to find a EPO mimetic which has the potential for oral activity.

A small molecule EPO mimetic has advantages over the natural protein. The immune response associated with large peptides is unlikely to occur with small molecules. In addition, the variety of pharmaceutical formulations that may be used with small molecules are technically unfeasible for proteins. Thus the use of relatively inert formulations for small molecules is possible. The most important advantage of small molecules is their potential for oral activity. Such an agent would ease administration, cost less and facilitate patient compliance.

Although compounds which mimic EPO are useful in stimulating red blood cell synthesis, there are diseases where the overproduction of red blood cells is a problem. Erythroleukemia and polysythemia vera are examples of such diseases. Since EPO is an agent responsible for the maturation of red blood cell precursors, an antagonist of EPO would have utility treating either of those diseases.

### SUMMARY OF THE INVENTION

The disclosed invention is drawn to a series of small molecules of Formula I: 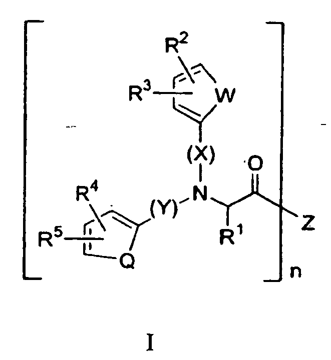 as defined in claim 1, which demonstrate competitive binding with the natural EPO receptor. As such, these compounds are potentially useful in the treatment of diseases or conditions associated with this receptor.

Thus, the present invention provides:
a compound of Formula I, for use as a medicament, wherein Formula I is as defined in claim 1;
a pharmaceutical composition, comprising a compound of Formula I, as defined in claim 12;
a method of making such a pharmaceutical composition, as defined in claim 13;
an *in vitro* method for modulating an EPO receptor, as defined in claim 14;
use of a compound of Formula I in the manufacture of a medicament, as defined in claim 15; and
certain compounds of Formula I *per se,* as defined in claim 16.

Certain compounds falling within the definition of Formula I given in claim 1 are disclosed in:
Kawase, Tetrahedron Letters, 35, 1, 149-152, 1994;
Hoffman & Tao, J. Org. Chem., 62, 2292-2297, 1997; and
Bonjoch et al., Tetrahedron: Asymmetry, 8,,18, 3143-3151, 1997.
These compounds have been disclaimed in claim 16.

Preferred features of the invention are recited in claims 2 to 11.

### DETAILED DESCRIPTION OF THE INVENTION

The terms used in describing the invention are commonly used and known to those skilled in the art. "Independently" means that when there are more than one substituent, the substitutents may be different. The term "alkyl" refers to straight, cyclic and branched-chain alkyl groups and "alkoxy" refers O-alkyl where alkyl is as defined supra. "Cbz" refers to benzyloxycarbonyl. "Boc" refers to t-butoxycarbonyl and "Ts" refers to toluenesulfonyl. "DCC" refers to 1,3-dicyclohexylcarbodiimide, "DMAP" refers to 4-N',N-dimethylaminopyridine and "HOBT" refers to 1-hydroxybenzotriazole hydrate. "Fmoc" refers to N-(9=fluoreaylmethoxycarbonyl), "DABCO" refers to 1,4-Diazabicyclo[2.2.2]octane, "EDCI" refers to 1-(3-dimethylaminopropyl)-3-ethylcarbodiimide, and "Dde" refers to 1-(4,4-dimethyl-2,6-dioxocyclohexylidene)ethyl. The side chains of α-amino acids refer to the substituents of the stereogenic carbon of an α-amino acid. For example if the amino acid is lysine, the side chain is 1-aminobutan-4-yl. The term natural amino acid refers to the 20 α-amino acids of the L configuration which are found in natural proteins. Unnatural α-amino acids include synthetic amino acids such as , -aminoadipic acid, 4-aminobutanoic acid, 6-aminohexanoic acid, -aminosuberic acid, 5-aminopentanoic acid, p-aminophenylalanine, -aminopimelic acid -carboxyglutamic acid, p-carboxyphenylalanine, carnitine, citrulline, - diaminopropionic acid, -diaminobutyric acid, homocitrulline, homoserine, and statine as well as D-configuration amino acids. The term "protectable group" refers to a hydroxy, amino, carboxy, carboxamide, guanidine, amidine or a thiol groups on an amino acid side. Compounds of the invention may be prepared by following general procedures known to those skilled in the art, and those set forth herein.

The compounds of the invention may be prepared by liquid phase organic synthesis techniques or by using amino acids which are bound to a number of known resins. The underlying chemistry, namely, acylation and alkylation reactions, peptide protection and deprotection reactions as well as peptide coupling reactions use similar conditions and reagents. The main distinction between the two methods is in the starting materials. While the starting materials for the liquid phase syntheses are the N-protected amino acids or the lower alkyl ester derivatives of the N-protected amino acids, the starting material for the resin syntheses are amino acids which are bound to resins by their carboxy terminuses.

### General Procedure For The Solid-Phase Synthesis Of Symmetrical N ,N -Disubstituted Amino Acids

### Scheme 1.

An equivalent of an N-Fmoc-protected amino acid which is bound to a resin **1a** is suspended in a suitable solvent such as DMF. This solvent is removed and the nitrogen protecting group (Fmoc) is removed by stirring the resin bound amino acid with an organic base, such as piperidine, and an addition portion of the solvent. A solution of about two to three equivalents of an appropriately substituted halide, **1b,** and a suitable base such DIEA is added to the resin bound amino acid and this mixture is shaken for 18-36 h. The resulting mixture is washed with several portions of a suitable solvent and is suspended and shaken in an acidic solution, such as 50% TFA/CH₂Cl₂, over several hours to cleave the acid from the resin and give the *N*-disubstituted amino acid **1c.**

By varying the resin bound amino acid **1a,** one may obtain many of the compounds of the invention. The following resin bound amino acids may be used in Scheme I: alanine, N-g-(4-methoxy-2,3,6-trimethylbenzenesulfonyl)arginine, -(4 - methyltrityl)asparagine, aspartic acid (-t-butyl ester), S-(trityl)cysteine, -(4-methyltrityl)glutamine, glutamic acid (-t-butyl ester), glycine, N-imidazolyl-(trityl)histidine, isoleucine, leucine, N- - (2-chlorobenzyloxycarbonyl)lysine, N--(t-butoxycarbonyl)lysine, methionine, phenylalanine, proline, O-(t-butyl)serine, O-(t-butyl)threonine, N-indolyl-(t-butoxycarbonyl)tryptophan, O-(t-butyl)tyrosine, valine, - alanine, -aminoadipic acid, 4-aminobutanoic acid,, 6-aminohexanoic acid, - aminosuberic acid, 5-aminopentanoic acid, p-aminophenylalanine, -aminopimelic acid -carboxyglutamic acid, p-carboxyphenylalanine, carnitine, citrulline, - diaminopropionic acid, -diaminobutyric acid, homocitrulline, homoserine, and statine. In addition, the choice of "W" and "X" can be varied by using known halide derivatives of **1b.** For example using benzylchloride, 2-chloromethylthiophene, or 2-chloromethylpyridine gives compounds of the invention where "W" is -CH=CH-, -S-, or -CH=N-, respectively. For variations in "X", the use of 2-chloroethylphenyl, 3-chloro-1-propenylbenzene, or benzeneacetyl chloride as **1b,** give compounds where Y is (CH₂)₂, -CH=CH-CH₂-, or -CH₂C(O)- respectively. Still further, Scheme 1 may be used to produce combinatorial mixtures of products. Using mixtures of resin bound amino acids, **1a,** with only one **1b** produces said combinatorial mixtures. Alternatively, using one amino acid **1a** with a mixture of **1b** as well as mixture of **1 a** with mixtures of **1b** gives a large range of combinatorial mixtures. 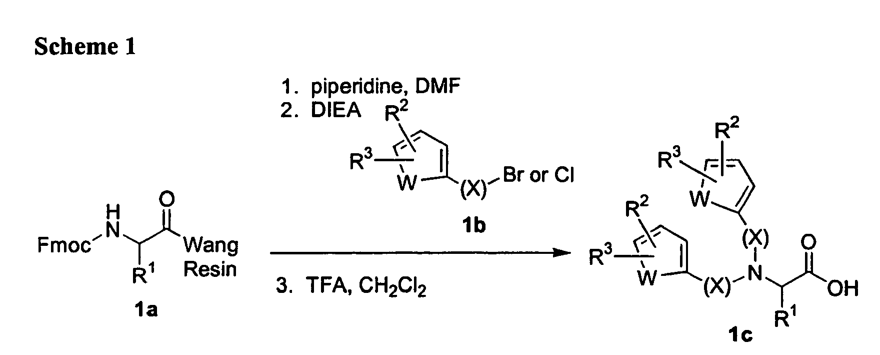 General Procedure For The Solid-Phase Synthesis Of Unsymmetrical N ,N -Disubstituted Amino Acids.

### Scheme 2, Step A

An equivalent of an N-Fmoc-protected amino acid which is bound to a resin **1a** is suspended in a suitable solvent such as DMF. This solvent is removed and the nitrogen protecting group (Fmoc) is removed by stirring the resin bound amino acid with an organic base, such as piperidine, and an addition portion of the solvent. Trimethyl orthoformate and an appropriately substituted aldehyde **2a** (5 equivalents) is added and the mixture is shaken under N₂ overnight. This mixture is treated with a suspension of NaBH(OAc)₃ (5 equivalents) in CH₂Cl₂ and shaken under N₂ overnight. After filtration and washing with a suitable solvent, the resulting product, resin bound N-monosubstituted amino acid **2b**, is rinsed with a suitable solvent and its identity is confirmed by MS and or HPLC analysis after treatmet of a portion of the resin with 50% TFA/CH₂Cl₂.

### Scheme 2, Step B

The resin **2b** is suspended in an appropriate solvent such as DMF and is filtered. The appropriately substituted alkyl or arylkyl halide, **2c,** and an appropriate base such as DIEA are added with some additional solvent and the mixture is shaken under N₂ for 18-36 h. The resin bound N, N -disubstituted amino acid, **2d,** is isolated from the suspension and the resin is cleaved with an acidic solution to give the free acid **2e.**

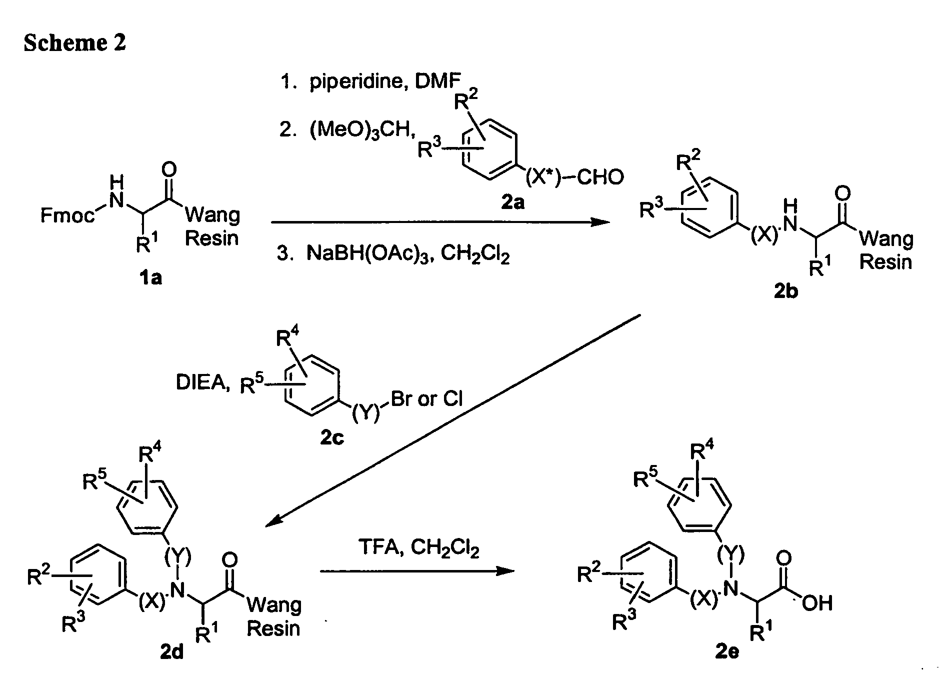

### Scheme 3, Step C

A resin bound amine, **2d,** where R⁴ is nitro, is suspended in a suitable solvent, such as DMF, and is filtered. This mixture is treated with SnCl₂ dihydrate in DMF and shaken under N₂ overnight. The solvent is removed and the resin is washed successive portions of a suitable solvent to give the resin bound compound **3a** where R⁴ is amino. The resin is suspended in a suitable solvent and is combined with an organic base, such as pyridine an appropriately substituted carboxylic acid anhydride, acid chloride, or sulfonyl chloride. The mixture is shaken under N₂ overnight and is filtered to give the resin bound amino acid **3b.** This material is treated with an acid and a suitable solvent to give the free amino acid **3b.**

### Scheme 3, Step D

The resin bound amine **3a** is treated with TMOF and an appropriately substituted aldehyde **3c** is added and the mixture is shaken under N₂ overnight. The resulting mixture is drained and treated with a suspension of NaBH(OAc)₃ in an appropriate solvent and this mixture is shaken under N₂ overnight. The resin bound 3-aralkylaminophenyl amino acid is identified my spectral techniques after cleavage to give the free acid **3d** as previously described.

### Scheme 3, Step E

Resin bound, **2d,** where R¹ is (CH₂)₄NH(Dde) is mixed with a suitable solvent, such as DMF, and shaken with successive portions of 2% solution of hydrazine hydrate in DMF over about 30 min. The resin is filtered and treated with a suitable solvent and a cyclic anhydride derivative **3e,** and a base such as DMAP and pyridine. This mixture is shaken under N₂ overnight and filtered to give the resin bound amine, **3f.** This material is identified by spectral techniques after cleavage to give the free acid **3f** as previously described.

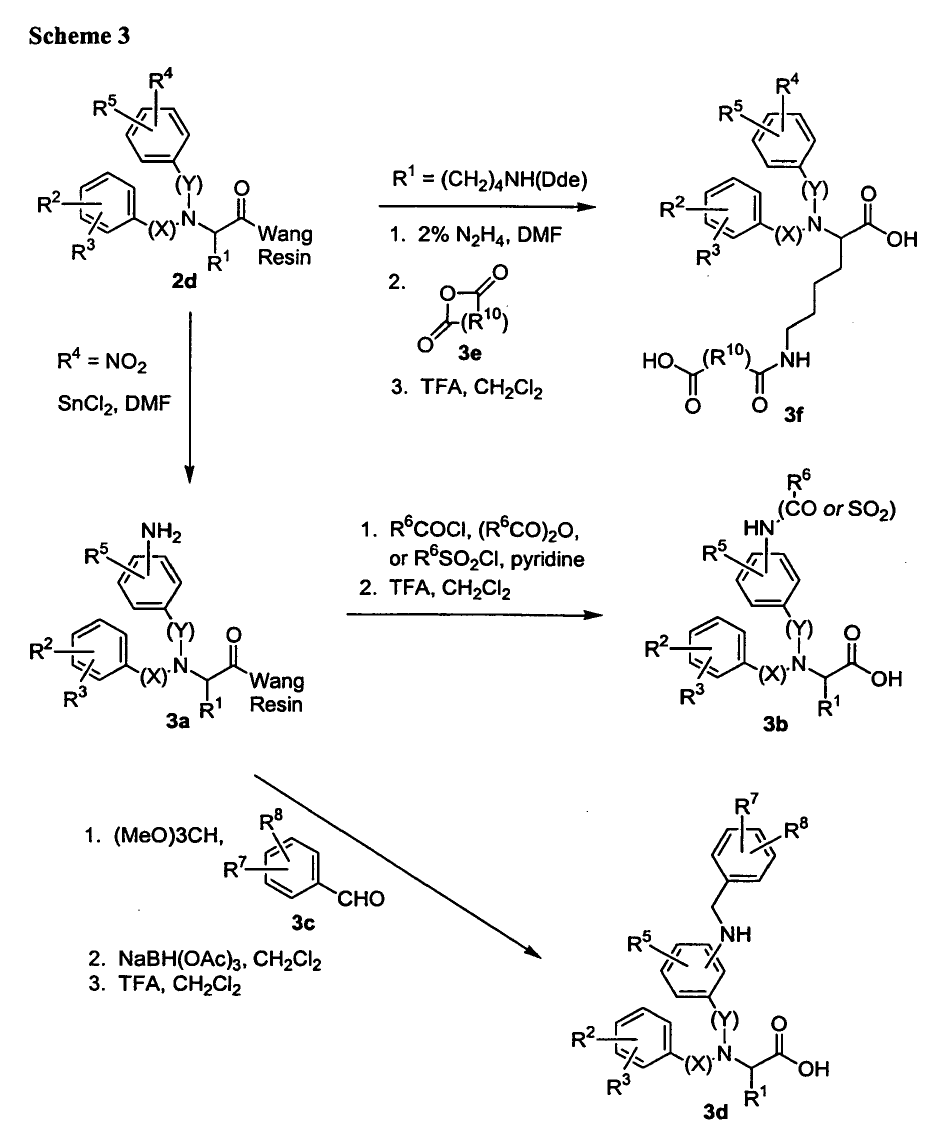

### Scheme 4, Step F

Resin bound **2b,** where R² is nitro is suspended in CH₂Cl₂ and is treated with an organic base, such as pyridine, and 9-fluorenylmethoxy chloride. This mixture is shaken under N₂ overnight, filtered and resuspended in a suitable solvent. This mixture is treated with SnCl₂ dihydrate in DMF and shaken under N₂ overnight. The solvent is removed and the resin is washed successive portions of a suitable solvent and filtered to give the resin bound compound **4a** where R² is amino. The resin **4a** is then suspended in a suitable solvent, such as CH₂Cl₂, and is combined with 0.4 mmol of pyridine and 0.25-0.4 mmol of the appropriately substituted carboxylic acid anhydride, acid chloride, or sulfonyl chloride. The mixture is shaken under N₂ overnight, filtered, and washed successively with three portions each of CH₂Cl₂ and MeOH. This resin is suspended in DMF, filtered, and shaken under N₂ with 5 mL of a 40% solution of piperidine in DMF. After 1 h, the solvent is drained and the resin was washed successively with three portions each of suitable solvents to give the resin bound **4b.** The identity of the compound was confirmed by spectral analysis after cleavage as previously described.

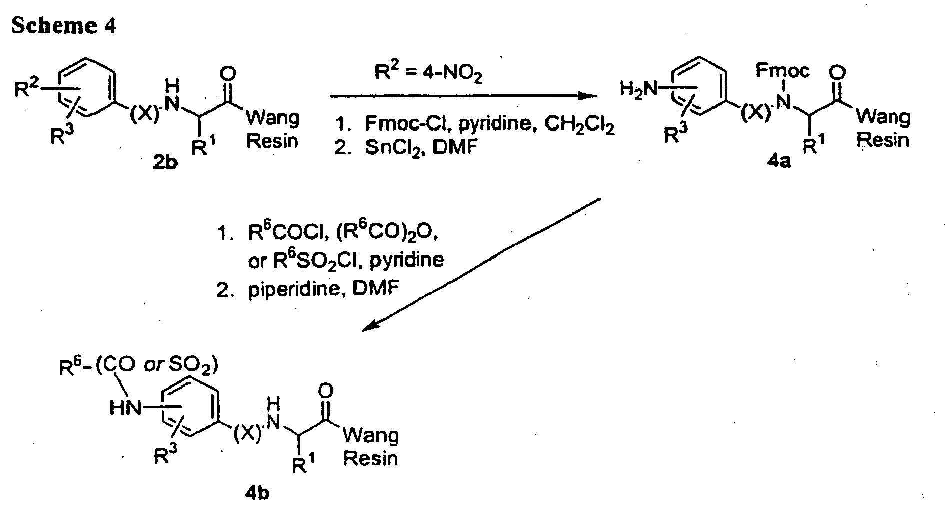

### Scheme 5

The resin **2b** (0.2 mmol) is suspended in CH₂Cl₂, filtered, and is resuspended in CH₂Cl₂. This suspension is treated with diethyl phosphonoacetic acid and diisopropylcarbodiimide or other suitable carbodiimide reagent, and the mixture is shaken under N₂ overnight. The solvent is drained and the resulting resin **5a** was washed successively with three portions each of CH₂Cl₂ and MeOH. The resin is suspended in DMF and filtered. A solution of the appropriately substituted aldehyde **5b** (0.6-1.0 mmol) in 3-5 mL of DMF, lithium bromide (0.6-1.0 mmol), and a suitable base such as DIEA or Et₃N (0.6-1.0 mmol) is added and the mixture is shaken under N₂ overnight. The solvent is removed and the resin is washed successively with three portions each of DMF, CH₂Cl₂, and MeOH. The identity of the resin bound substituted amino acid **5c** was confirmed spectral techniques. The resin bound material may be treated with 50% TFA/CH₂Cl₂ over 1-1.5 h, to give the acid **5c.**

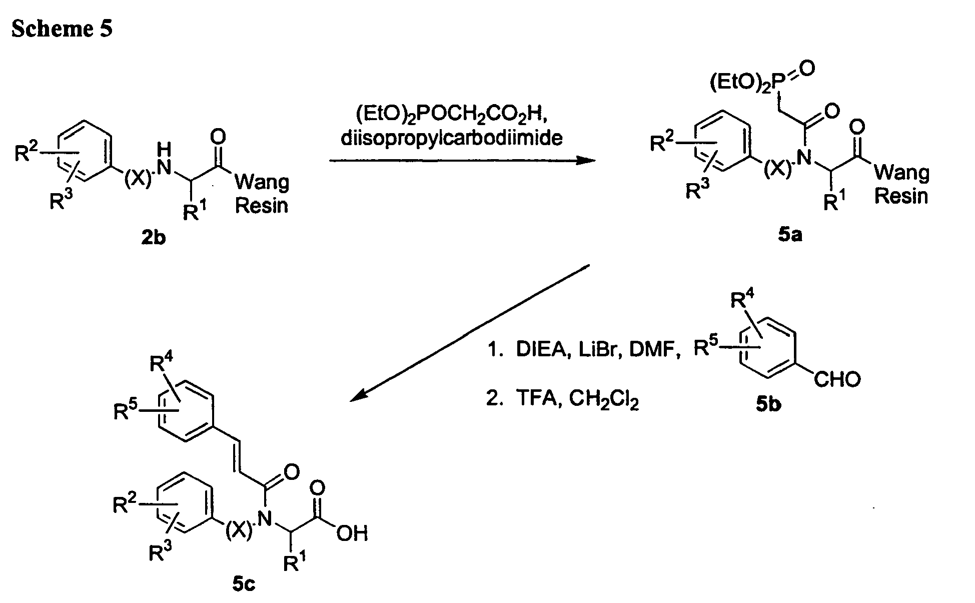

### Scheme 6

To prepare compounds where n is 2 and Z is NH(CH₂)ₛNH, products of Schemes 1-5 may be used in Scheme 6. Treatment of two equivalents of the substituted amino acid **1c** with an equivalent of the diamine **6a,** in the presence of HOBT and a peptide coupling agent such as EDCI and a base such as DIEA at room temperature over 16 h gives the dimer **6b.**

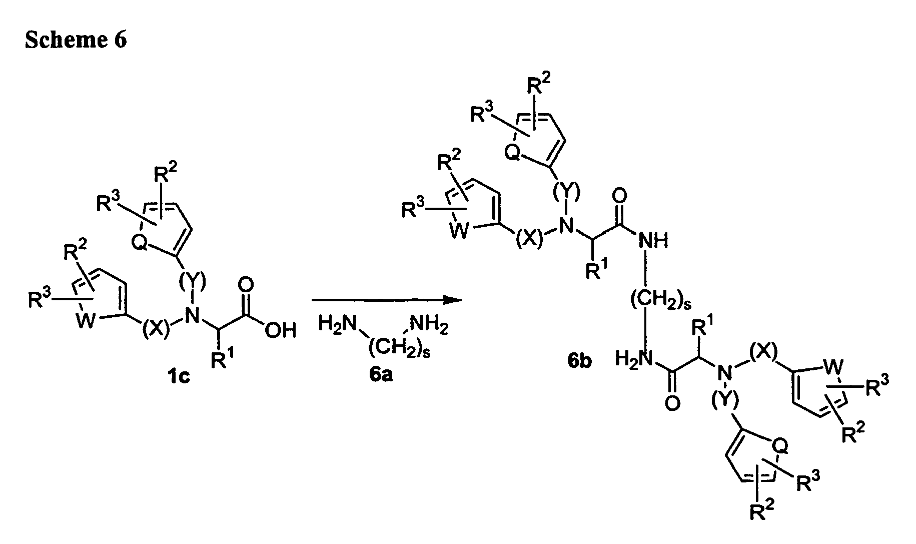

### General Procedure For The Solution-Phase Synthesis Of Symmetrical N ,N -Disubstituted Amino Acids

### Scheme 7, Step A

A solution of of amino acid ester **7a,** an appropriately substituted halide derivitive **1b,** and an appropriate base such as DIEA, Na₂CO₃, or Cs₂CO₃ in a suitable solvent, such as DMF, is heated at 50-100 °C under N₂ overnight, or until the starting material is exhausted, to give a mixture of the di and mono-substituted amines, **7b** and **7c** respectively. If the side chains of R¹ contain acid cleavable protecting groups, those groups may be cleaved by treatment with 30-80% TFA/CH₂Cl₂. Esters **7b** and **7c** may be independently converted to the corresponding acids **7d** and **7e** by hydrolysis with an appropriate base such as aqueous NaOH.

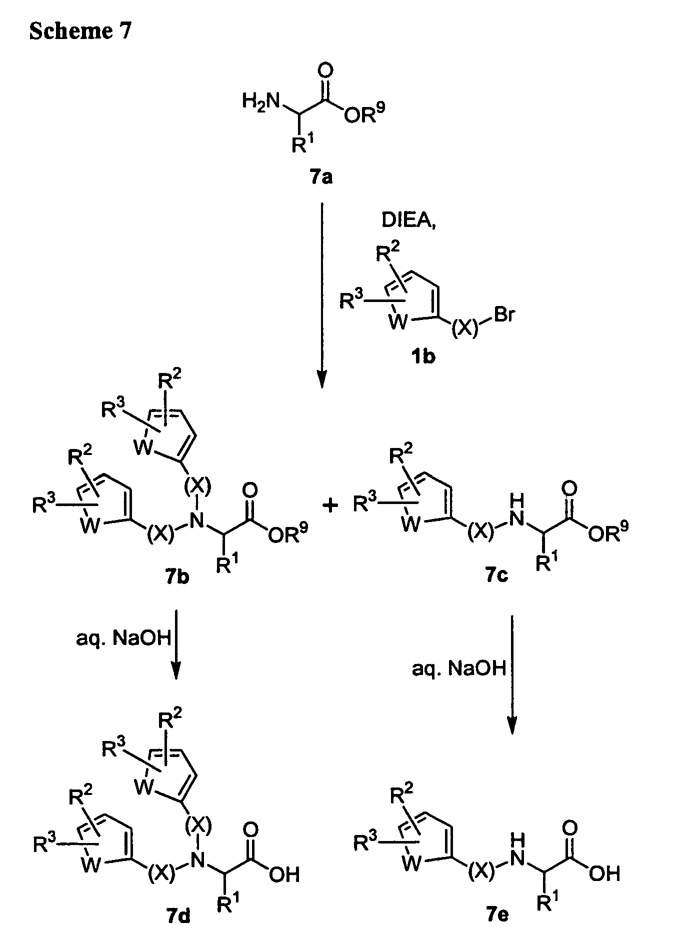

### General Procedure For The Solution-Phase Synthesis Of Unsymmetrical N ,N -Disubstituted Amino Acids

### Scheme 8, Step A

A solution of 1 mmol of amino acid ester **8a** (or the corresponding HCl salt and 1.1 mmol of DIEA) and 1-1.5 mmol of the appropriately substituted aldehyde **2a** in 3-5 mL of trimethyl orthoformate was stirred at room temperature under N₂ overnight. The solution was either concentrated and used directly for the next reaction, or was partitioned between EtOAc and water, washed with brine, dried over Na₂SO₄, and concentrated to give crude product, which was purified by MPLC to give mono-substituted product **8b.**

### Scheme 8, Step B

Amino ester **8b** was dissolved in DMF, combined with 1.1-1.5 mmol of the appropriately substituted chloride or bromide **2c,** and heated at 50-100 °C overnight. The reaction mixture was cooled and partitioned between water and EtOAc. The organic layer was washed three times with water and once with brine, dried over Na₂SO₄, and concentrated. The crude product was purified by MPLC to give pure **8c.** For examples of **8c** wherein the side chain R¹ contained an acid-cleavable protecting group such as t-butylcarbamate, t-butyl ester, or t-butyl ether, **8c** was stirred in 30-80% TFA/CH₂Cl₂ for 1-3 h. The reaction mixture was concentrated and optionally dissolved in HOAc and freeze-dried to give the deprotected form of **8c**. For examples of **8c** where R⁹ was equal to t-butyl, **8c** was stirred in 30-80% TFA/CH₂Cl₂ for 1-3 h and treated as described above to give acid **8d**. For examples of **8c** where R⁹ was equal to methyl, ethyl, or other primary or secondary alkyl esters, **8c** was stirred with with 1-2 mmol of aqueous LiOH, NaOH, or KOH in MeOH, EtOH, or THF at 20-80 °C until TLC indicated the absence of **8c**. The solution was acidified to pH 4-5 with aqueous citric acid or HCl and was extracted with CH₂Cl₂ or EtOAc. The organic solution was washed with brine, dried over Na₂SO₄, and concentrated to give **8d.**

### Scheme 8, Step C

For examples of amino acid ester **8c** where R¹ = (CH₂)₄NHBoc, **8c** (1 mmol) was stirred in 30-80% TFA/CH₂Cl₂ for 1-3 h. The reaction mixture was concentrated to provide **8e** as the TFA salt. Optionally, the TFA salt was dissolved in CH₂Cl₂ or EtOAc and washed with aqueous NaOH or Na₂CO₃, dried over Na₂SO₄, and concentrated to give **8e** as the free base.

### Scheme 8, Step D

A solution of 1 mmol of **8e,** 1-4 mmol of an appropriate base such as DIEA, and 1-2 mmol of the appropriately substituted cyclic anhydride **3e** was stirred in CH₂Cl₂ or DMF under N₂ overnight. The resulting mixture was diluted with CH₂Cl₂ or EtOAc and washed with aqueous HCl, water, and brine, was dried over Na₂SO₄, and concentrated to provide **8f.** Alternatively, 1 mmol of **8e,** 1-4 mmol of an appropriate base such as DIEA, and 1-2 mmol of the appropriately substituted carboxylic acid anhydride (R¹¹CO)₂O or acid chloride R¹¹COCl was stirred in CH₂Cl₂ or DMF under N₂ overnight and worked up as above to provide **8g.** Alternatively, 1 mmol of **8e,** 1-4 mmol of an appropriate base such as DIEA, and 1-2 mmol of the appropriately substituted isocyanate R¹²NCO was stirred in CH₂Cl₂ or DMF under N₂ overnight and worked up as above to provide **8h.**

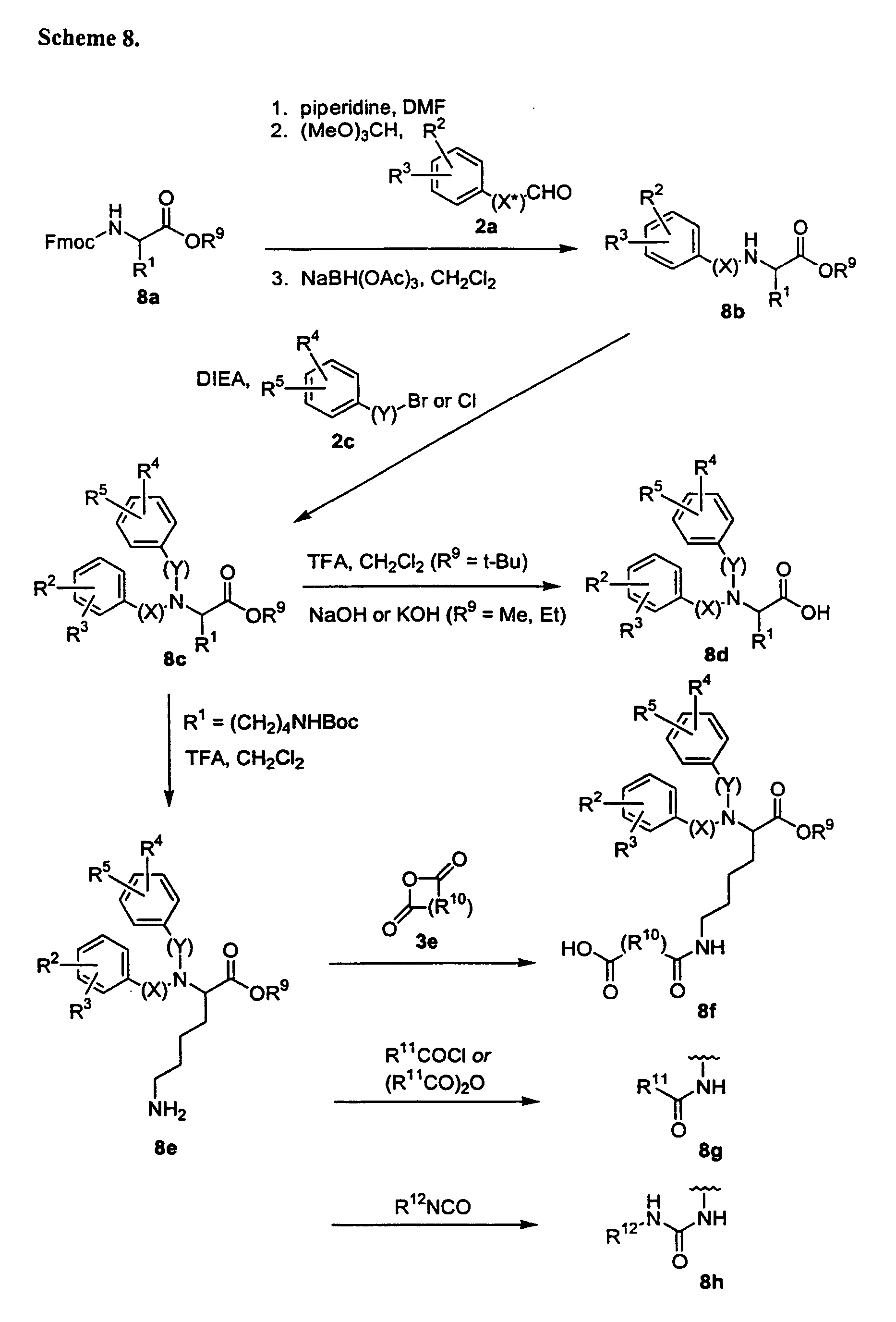

### Scheme 9, Step A

For examples of **8c** where R⁵ = NO₂, a solution of 1 mmol of **8c** (where R², R³, R⁴, or) and 10-12 mmol of SnCl₂ dihydrate was stirred in MeOH, EtOH, or DMF at 20-80 °C for 0.5-24 h under N₂. The solution was taken to room temperature and poured into aqueous Na₂CO₃ with rapid stirring. The resulting mixture was extracted with EtOAc or CH₂Cl₂ and the organic extracts were washed with brine, dried over Na₂SO₄, and concentrated to give the aminophenyl product **9a,** which was purified by MPLC or used without further purification.

### Scheme 9, Step B

A solution of 1 mmol of aminophenyl compound **9a** and 1-1.5 mmol of the appropriately substituted aldehyde **2a** in 3-5 mL of trimethyl orthoformate was stirred at room temperature under N₂ overnight. The solution was either concentrated and used directly for the next reaction, or was partitioned between EtOAc and water, washed with brine, dried over Na₂SO₄, and concentrated to give crude product, which was purified by MPLC to give **9b.** For examples of **9b** wherein the side chain R¹ or R⁹ contained an acid-cleavable protecting group such as t-butylcarbamate, t-butyl ester, or t-butyl ether, **9b** was stirred in 30-80% TFA/CH₂Cl₂ for 1-3 h. The reaction mixture was concentrated and optionally dissolved in HOAc and freeze-dried to give the deprotected form of **9b.**

### Scheme 9, Step C

A solution of 1 mmol of 3-aminophenyl compound **9a,** 1.1-2 mmol of pyridine, and 1-1.5 mmol of the appropriately substituted acid chloride, acid anhydride, or sulfonyl chloride in 3-5 mL of CH₂Cl₂ or ClCH₂CH₂Cl was stirred at room temperature under N₂ overnight. The solution was partitioned between EtOAc and water, washed with water, saturated aqueous NaHCO₃, and brine, dried over Na₂SO₄, and concentrated to give crude product which was optionally purified by MPLC to give amide or sulfonamide **9c.** For examples of **9c** wherein the side chain R¹ or R⁹ contained an acid-cleavable protecting group such as t-butylcarbamate, t-butyl ester, or t-butyl ether, **9c** was stirred in 30-80% TFA/CH₂Cl₂ for 1-3 h. The reaction mixture was concentrated and optionally dissolved in HOAc and freeze-dried to give the deprotected form of **9c.**

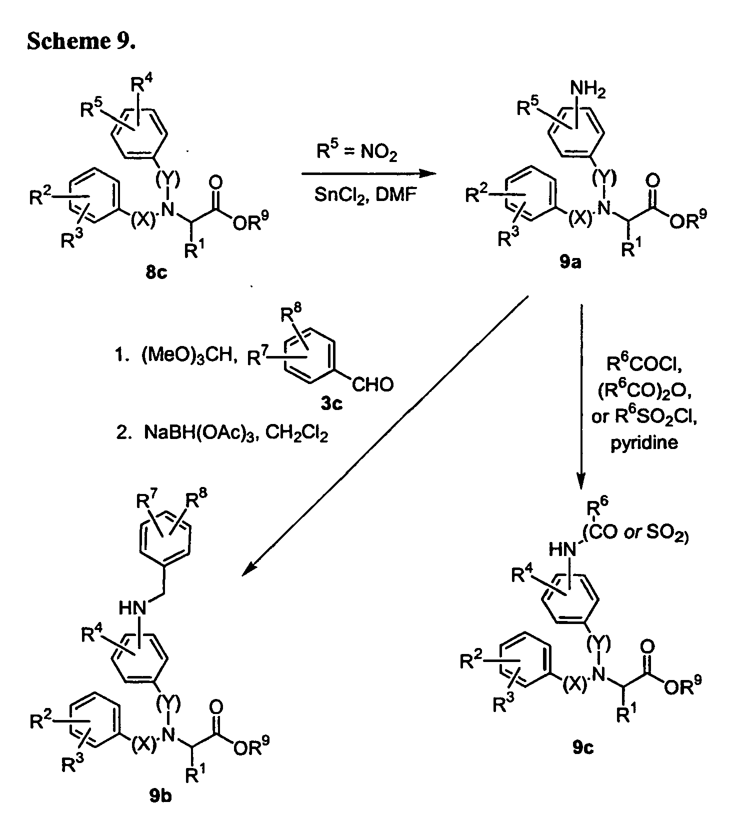

### General Procedure For The Solution-Phase Synthesis Of Symmetrical N ,N -Disubstituted Amino Amides And Their Dimers and Trimers

### Scheme 10, Step A

A solution of 1 mmol of N-Cbz-protected amino acid **10a** and the appropriate amine (ZH, 1 mmol), diamine (ZH₂, 0.5 mmol), or triamine (ZH₃ 0.33 mmol), was treated with 1.1 mmol of HOBt, 1.1 mmol of DIEA, and 2.1 mmol of EDCI in 3-6 mL of CH₂Cl₂ or DMF. [Alternatively, 1 mmol of the pentafluorophenyl ester or N-hydroxysuccinimide ester of **10a** was mixed with the appropriate portion of amine (ZH), diamine (ZH₂), or triamine (ZH₃) in 3-6 mL of DMF.] The solution was stirred at room temperature under N₂ for 12-24 h, and EtOAc was added. The organic solution was washed with 5% aqueous citric acid, water, saturated NaHCO₃, and brine, dried over Na₂SO₄, and concentrated. The crude product was optionally purified by MPLC to afford amide **10b.** Compound **10b** was stirred in 30-80% TFA/CH₂Cl₂ for 1-3 h. The reaction mixture was concentrated to provide the TFA salt which was dissolved in CH₂Cl₂ or EtOAc and washed with aqueous NaOH or Na₂CO₃, dried over Na₂SO₄, and concentrated to give **10c** as the free base.

### Scheme 10, Step B

A solution of 1 mmol of amino acid ester **10c** (n = 1), 2.5-3 mmol of the appropriately substituted chloride or bromide **2c,** and 2.5-3 mmol of an appropriate base such as DIEA, Na₂CO₃, or Cs₂CO₃ in 3-5 mL of DMF was heated at 50-100 °C under N₂ for 18-24 h. (For examples of **10c** where n = 2 or 3, the amounts of **2c** and base were increased by two- or three-fold, respectively.) The reaction mixture was cooled and partitioned between water and EtOAc. The organic layer was washed three times with water and once with brine, dried over Na₂SO₄, and concentrated. The crude product was purified by MPLC to give pure amide **10d.**

Alternatively, a solution of 1 mmol of amino acid ester **10c** (n = 1), 2.5-3 mmol of the appropriately substituted aldehyde **2a,** and 2.5-3 mmol of borane-pyridine complex in 3-5 mL of DMF or EtOH was stirred at room temperature under N₂ for 3-5 days.. (For examples of **10c** where n = 2 or 3, the amounts of **2c** and borane-pyridine complex were increased by two- or three-fold, respectively.) The mixture was concentrated to dryness and was partitioned between water and CH₂Cl₂, washed with brine, dried over Na₂SO₄, and concentrated. The crude product was purified by MPLC to give pure amide **10d.**

### Scheme 10, Step C

For examples of **10d** where R¹ = CH₂CH₂CO₂-t-Bu or CH₂CO₂-t-Bu, **10d** was stirred in 30-80% TFA/CH₂Cl₂ for 1-24 h. The reaction mixture was concentrated and optionally dissolved in HOAc and freeze-dried to give acid **10e.**

### Scheme 10, Step D

For examples of **10d** where R¹ is equal to (CH₂)₄NHBoc, **10d** was stirred in 30-80% TFA/CH₂Cl₂ for 1-24 h. The reaction mixture was concentrated and optionally dissolved in HOAc and freeze-dried to give amine **10f** as the TFA salt which was optionally dissolved in CH₂Cl₂ or EtOAc, washed with aqueous NaOH or Na₂CO₃, dried over Na₂SO₄, and concentrated to give **10f** as the free base.

### Scheme 10, Step E

A solution of 1 mmol **of 10f,** 1-4 mmol of an appropriate base such as DIEA, and 1-2 mmol of the appropriately substituted cyclic anhydride **3e** was stirred in CH₂Cl₂ or DMF under N₂ overnight. The resulting mixture was diluted with CH₂Cl₂ or EtOAc and washed with aqueous HCl, water, and brine, was dried over Na₂SO₄, and concentrated to provide acid **10g.** Alternatively, 1 mmol of **10f,** 1-4 mmol of an appropriate base such as DIEA, and 1-2 mmol of the appropriately substituted carboxylic acid anhydride (R¹¹CO)₂O or acid chloride R¹¹COCl was stirred in CH₂Cl₂ or DMF under N₂ overnight and worked up as above to provide **10h.** Alternatively, 1 mmol of **8e,** 1-4 mmol of an appropriate base such as DIEA, and 1-2 mmol of the appropriately substituted isocyanate R¹²NCO was stirred in CH₂Cl₂ or DMF under N₂ overnight and worked up as above to provide **10i.**

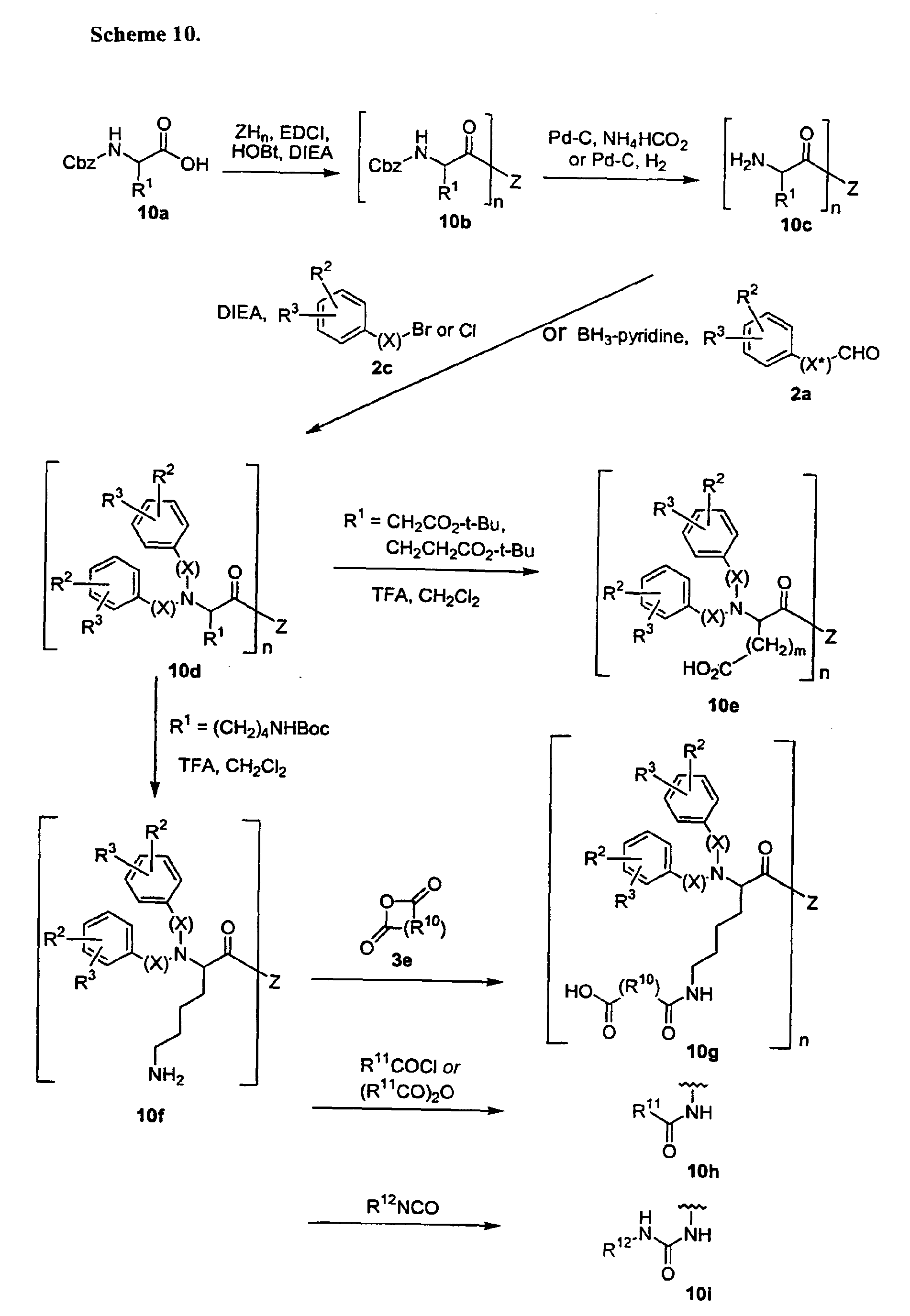

Although the claimed compounds are useful as competitive binders to the EPO receptor, some compounds are more active than others and are either preferred or particularly preferred.

The preferred compounds of the invention include: 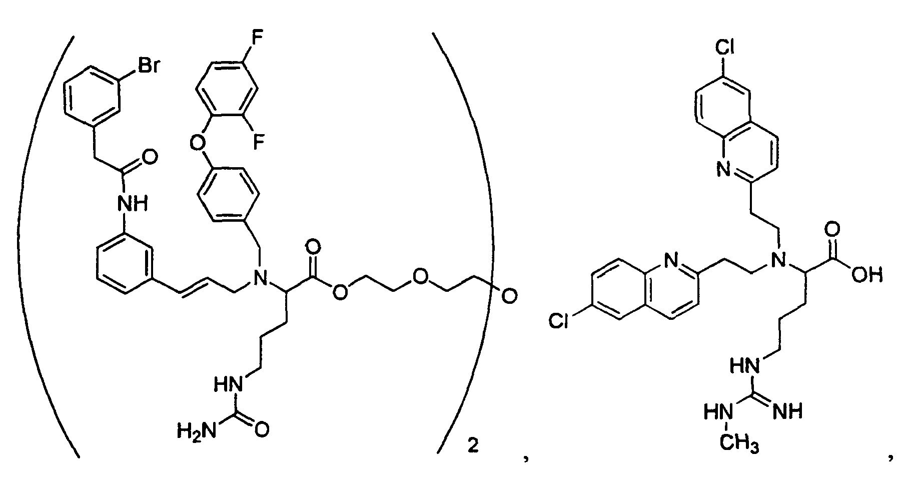 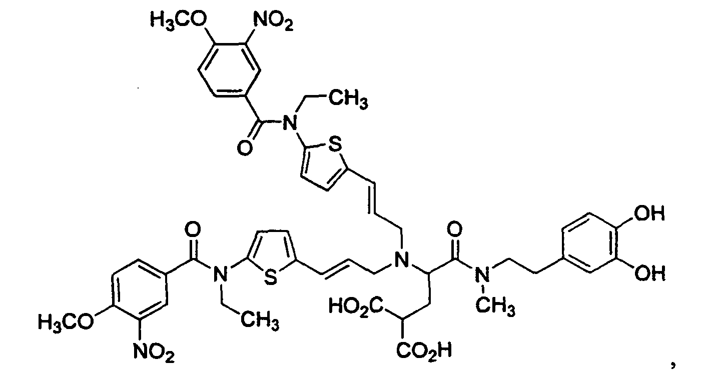 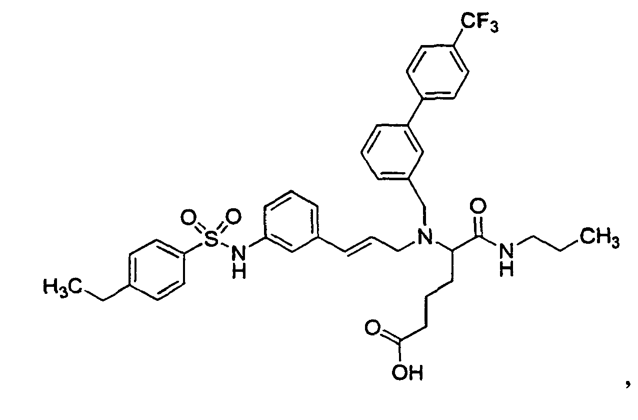 and

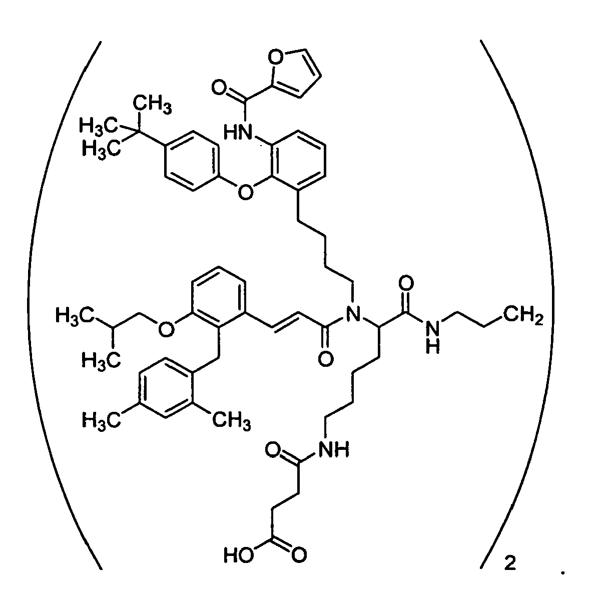

The particularly preferred "R¹ "s are the side chain of lysine, ornithine, arginine, aspartic acid, glutamic acid, glutamine, cysteine, methionine, serine, and threonine.

The particularly preferred "R² and R³" s are phenoxy, substituted phenoxy, benzyloxy, and substituted benzyloxy.

The particularly preferred "R⁴ and R⁵" s are phenoxy, substituted phenoxy, benzyloxy, and substituted benzyloxy.

The particularly preferred "W" is -CH=CH-

The particularly preferred "Q" is -CH=CH-

The particularly preferred "X" are C₁₋₅alkenyl and CH₂.

The particularly preferred "Y" are C₁₋₅alkenyl and CH₂.

The particularly preferred "n" are 1 and 2.

The particularly preferred "Z" are hydroxy, methoxy, phenethylamino, substituted phenethylamino, and -NH(CH₂)₂O(CH₂)₂O(CH₂)₂NH-.

Pharmaceutically useful compositions containing the compounds of the present invention, may be formulated according to known methods such as by the admixture of a pharmaceutically acceptable carrier. Examples of such carriers and methods of formulation may be found in Remington's Pharmaceutical Sciences. To form a pharmaceutically acceptable composition suitable for effective administration, such compositions will contain an effective amount of the compound of the present invention.

Therapeutic or diagnostic compositions of the invention are administered to an individual in amounts sufficient to treat or diagnose disorders in which modulation of EPO receptor-related activity is indicated. The effective amount may vary according to a variety of factors such as the individual's condition, weight, sex and age. Other factors include the mode of administration. The pharmaceutical compositions may be provided to the individual by a variety of routes such as subcutaneous, topical, transdermal, oral and parenteral.

The term "chemical derivative" describes a molecule that contains additional chemical moieties which are not normally a part of the base molecule. Such moieties may improve the solubility, half-life, absorption, etc. of the base molecule. Alternatively the moieties may attenuate undesirable side effects of the base molecule or decrease the toxicity of the base molecule. Examples of such moieties are described in a variety of texts, such as Remington's Pharmaceutical Sciences.

Compounds disclosed herein may be used alone at appropriate dosages defined by routine testing in order to obtain optimal inhibition of the EPO receptor or its activity while minimizing any potential toxicity. In addition, co-administration or sequential administration of other agents may be desirable.

The present invention also has the objective of providing suitable topical, transdermal, oral, systemic and parenteral pharmaceutical formulations for use in novel methods of treatment. The compositions containing compounds according to this invention as the active ingredient for use in the modulation of EPO receptors can be administered in a wide variety of therapeutic dosage forms in conventional vehicles for administration. For example, the compounds or modulators can be administered in such oral dosage forms as tablets, capsules (each including timed release and sustained release formulations), pills, powders, granules, elixirs, tinctures, solutions, suspensions, syrups and emulsions, or by transdermal delivery or injection. Likewise, they may also be administered in intravenous (both bolus and infusion), intraperitoneal, subcutaneous, topical with or without occlusion, transdermal, or intramuscular form, all using forms well known to those of ordinary skill in the pharmaceutical arts. The compounds of the present invention may be delivered by a wide variety of mechanisms, including but not limited to, transdermal delivery, or injection by needle or needle-less injection means. An effective but non-toxic amount of the compound desired can be employed as an EPO receptor modulating agent.

The daily dosage of the products may be varied over a wide range from 0.01 to 1,000 mg per patient, per day. For oral administration, the compositions are preferably provided in the form of scored or unscored tablets containing 0.01, 0.05, 0.1, 0.5, 1.0, 2.5, 5.0, 10.0, 15.0, 25.0, and 50.0 milligrams of the active ingredient for the symptomatic adjustment of the dosage to the patient to be treated. An effective amount of the drug is ordinarily supplied at a dosage level of from about 0.0001 mg/kg to about 100 mg/kg of body weight per day. The range is more particularly from about 0.001 mg/kg to 10 mg/kg of body weight per day. The dosages of the EPO receptor modulators are adjusted when combined to achieve desired effects. On the other hand, dosages of these various agents may be independently optimized and combined to achieve a synergistic result wherein the pathology is reduced more than it would be if either agent were used alone.

Advantageously, compounds or modulators of the present invention may be administered in a single daily dose, or the total daily dosage may be administered in divided doses of two, three or four times daily. Furthermore, compounds or modulators for the present invention can be administered in intranasal form via topical use of suitable intranasal vehicles, or via transdermal routes, using those forms of transdermal skin patches well known to those of ordinary skill in that art. To be administered in the form of a transdermal delivery system, the dosage administration will, of course, be continuous rather than intermittent throughout the dosage regimen.

For combination treatment with more than one active agent, where the active agents are in separate dosage formulations, the active agents can be administered concurrently, or they each can be administered at separately staggered times.

The dosage regimen utilizing the compounds or modulators of the present invention is selected in accordance with a variety of factors including type, species, age, weight, sex and medical condition of the patient; the severity of the condition to be treated; the route of administration; the renal and hepatic function of the patient; and the particular compound thereof employed. A physician or veterinarian of ordinary skill can readily determine and prescribe the effective amount of the drug required to prevent, counter or arrest the progress of the condition. Optimal precision in achieving concentrations of drug within the range that yields efficacy without toxicity requires a regimen based on the kinetics of the drug's availability to target sites. This involves a consideration of the distribution, equilibrium, and elimination of a drug.

In the methods of treatment, the compounds or modulators herein described in detail can form the active ingredient, and are typically administered in admixture with suitable pharmaceutical diluents, excipients or carriers (collectively referred to herein as "carrier" materials) suitably selected with respect to the intended form of administration, that is, oral tablets, capsules, elixirs, syrups and the like, and consistent with conventional pharmaceutical practices.

For instance, for oral administration in the form of a tablet or capsule, the active drug component can be combined with an oral, non-toxic pharmaceutically acceptable inert carrier such as ethanol, glycerol, water and the like. Moreover, when desired or necessary, suitable binders, lubricants, disintegrating agents and coloring agents can also be incorporated into the mixture. Suitable binders include, without limitation, starch, gelatin, natural sugars such as glucose or beta-lactose, corn sweeteners, natural and synthetic gums such as acacia, tragacanth or sodium alginate, carboxymethylcellulose, polyethylene glycol, waxes and the like. Lubricants used in these dosage forms include, without limitation, sodium oleate, sodium stearate, magnesium stearate, sodium benzoate, sodium acetate, sodium chloride and the like. Disintegrators include, without limitation, starch, methyl cellulose, agar, bentonite, xanthan gum and the like.

For liquid forms the active drug component can be combined in suitably flavored suspending or dispersing agents such as the synthetic and natural gums, for example, tragacanth, acacia, methyl-cellulose and the like. Other dispersing agents which may be employed include glycerin and the like. For parenteral administration, sterile suspensions and solutions are desired. Isotonic preparations which generally contain suitable preservatives are employed when intravenous administration is desired.

Topical preparations containing the active drug component can be admixed with a variety of carrier materials well known in the art, such as, e.g., alcohols, aloe vera gel, allantoin, glycerine, vitamin A and E oils, mineral oil, PPG2 myristyl propionate, and the like, to form, e.g., alcoholic solutions, topical cleansers, cleansing creams, skin gels, skin lotions, and shampoos in cream or gel formulations.

The compounds or modulators of the present invention can also be administered in the form of liposome delivery systems, such as small unilamellar vesicles, large unilamellar vesicles and multilamellar vesicles. Liposomes can be formed from a variety of phospholipids, such as cholesterol, stearylamine or phosphatidylcholines.

Compounds of the present invention may also be delivered by the use of monoclonal antibodies as individual carriers to which the compound molecules are coupled. The compounds or modulators of the present invention may also be coupled with soluble polymers as targetable drug carriers. Such polymers can include polyvinyl-pyrrolidone, pyran copolymer, polyhydroxypropylmethacryl-amidephenol, polyhydroxy-ethylaspartamidephenol, or polyethyl-eneoxidepolylysine substituted with palmitoyl residues. Furthermore, the compounds or modulators of the present invention may be coupled to a class of biodegradable polymers useful in achieving controlled release of a drug, for example, polylactic acid, polyepsilon caprolactone, polyhydroxy butyric acid, polyorthoesters, polyacetals, polydihydro-pyrans, polycyanoacrylates and cross-linked or amphipathic block copolymers of hydrogels, and other suitable polymers known to those skilled in the art.

For oral administration, the compounds or modulators may be administered in capsule, tablet, or bolus form or alternatively they can be mixed in the animals feed. The capsules, tablets, and boluses are comprised of the active ingredient in combination with an appropriate carrier vehicle such as starch, talc, magnesium stearate, or di-calcium phosphate. These unit dosage forms are prepared by intimately mixing the active ingredient with suitable finely-powdered inert ingredients including diluents, fillers, disintegrating agents, and/or binders such that a uniform mixture is obtained. An inert ingredient is one that will not react with the compounds or modulators and which is non-toxic to the animal being treated. Suitable inert ingredients include starch, lactose, talc, magnesium stearate, vegetable gums and oils, and the like. These formulations may contain a widely variable amount of the active and inactive ingredients depending on numerous factors such as the size and type of the animal species to be treated and the type and severity of the infection. The active ingredient may also be administered as an additive to the feed by simply mixing the compound with the feedstuff or by applying the compound to the surface of the feed. Alternatively the active ingredient may be mixed with an inert carrier and the resulting composition may then either be mixed with the feed or fed directly to the animal. Suitable inert carriers include corn meal, citrus meal, fermentation residues, soya grits, dried grains and the like. The active ingredients are intimately mixed with these inert carriers by grinding, stirring, milling, or tumbling such that the final composition contains from 0.001 to 5% by weight of the active ingredient.

The compounds or modulators may alternatively be administered parenterally via injection of a formulation consisting of the active ingredient dissolved in an inert liquid carrier. Injection may be either intramuscular, intraruminal, intratracheal, or subcutaneous, either by needle or needle-less means. The injectable formulation consists of the active ingredient mixed with an appropriate inert liquid carrier. Acceptable liquid carriers include the vegetable oils such as peanut oil, cotton seed oil, sesame oil and the like as well as organic solvents such as solketal, glycerol formal and the like. As an alternative, aqueous parenteral formulations may also be used. The vegetable oils are the preferred liquid carriers. The formulations are prepared by dissolving or suspending the active ingredient in the liquid carrier such that the final formulation contains from 0.005 to 10% by weight of the active ingredient.

Topical application of the compounds or modulators is possible through the use of a liquid drench or a shampoo containing the instant compounds or modulators as an aqueous solution or suspension. These formulations generally contain a suspending agent such as bentonite and normally will also contain an antifoaming agent. Formulations containing from 0.005 to 10% by weight of the active ingredient are acceptable. Preferred formulations are those containing from 0.01 to 5% by weight of the instant compounds or modulators.

The compounds of Formula I may be used in pharmaceutical compositions to treat patients (humans and other mammals) with disorders or conditions associated with the production of erythropoietin or modulated by the EPO receptor. The compounds can be administered in the manner of the commercially available product or by any oral or parenteral route (including but not limited to, intravenous, intraperitoneal, intramuscular, subcutaneous, dermal patch), where the preferred route is by injection. When the method of administration is intravenous infusion, compound of Formula I may be administered in a dose range of about 0.01 to 1 mg/kg/min. For oral administration, the dose range is about 0.1 to 100 mg/kg.

The pharmaceutical compositions can be prepared using conventional pharmaceutical excipients and compounding techniques. Oral dosage forms may be used and are elixirs, syrups, capsules, tablets and the like. Where the typical solid carrier is an inert substance such as lactose, starch, glucose, methyl cellulose, magnesium stearate, dicalcium phosphate, mannitol and the like; and typical liquid oral excipients include ethanol, glycerol, water and the like. All excipients may be mixed as needed with disintegrants, diluents, granulating agents, lubricants, binders and the like using conventional techniques known to those skilled in the art of preparing dosage forms. Parenteral dosage forms may be prepared using water or another sterile carrier.

Typically the compounds of Formula I are isolated as the free base, however when possible pharmaceutically acceptable salts can be prepared. Examples of such salts include hydrobromic, hydroiodic, hydrochloric, perchloric, sulfuric, maleic, fumaric, malic, tartaric, citric, benzoic, mandelic, methanesulfonic, hydroethanesulfonic, benzenesulfonic, oxalic, pamoic, 2-naphthalenesulfonic, p-toluenesulfonic, cyclohexanesulfamic and saccharic.

In order to illustrate the invention the following examples are included. These examples do not limit the invention. They are only meant to suggest a method of practising the invention. Those knowledgeable in chemical synthesis and the treatment of EPO related disorders may find other methods of practising the invention. However those methods are deemed to be within the scope of this invention.

### BIOLOGICAL EXAMPLES

The compounds of the invention were evaluated for the ability to compete with EPO in the following immobilized EPO receptor preparation (EBP-Ig, EPO binding protein-Ig).

EBP-Ig fusion protein (as disclosed in WO97/27219) was purified by affinity chromatography from the conditioned media of NSO cells engineered to express a recombinant gene construct which functionally joined the N-terminal 225 amino acids of the human EPO receptor and an Ig heavy chain as described herein. The interaction of biotin and streptavidin is frequently employed to capture and effectively immobilize reagents useful in assay protocols and has been employed here as a simple method to capture and immobilize EBP-Ig. EBP-Ig is initially randomly modified with an amine reactive derivative of biotin to produce biotinylated-EBP-Ig. Use of streptavidin coated plates allows the capture of the biotinylated EBP-Ig on the surface of a scintillant impregnated coated well (Flash plates, NEN-DuPont). Upon binding of [¹²⁵I]EPO to the ligand binding domain, specific distance requirements are satisfied and the scintillant is induced to emit light in response to the energy emitted by the radioligand. Unbound radioligand does not produce a measurable signal because the energy from the radioactive decay is too distant from the scintillant. The amount of light produced was quantified to estimate the amount of ligand binding. The specific assay format was suitable for the multi-well plate capacity of a Packard TopCount Microplate Scintillation counter. Compounds which were capable of reducing the amount of detected signal through competitive binding with the radioligand were identified.

Biotinylated EBP-Ig was prepared as follows. EBP-Ig (3 mL, OD₂₈₀ 2.9) was exchanged into 50 mM sodium bicarbonate, pH 8.5 using a Centriprep 10 ultrafiltration device. The final volume of the exchanged protein was 1.2 mL (OD₂₈₀ 2.6, representing about 2 mg total protein). 10 L of a 4 mg/ml solution of NHS-LC-Biotin (Pierce) was added and the reaction mixture placed on ice in the dark for two hours. Unreacted biotin was removed by exchange of the reaction buffer into PBS in a Centriprep 10 device and the protein reagent aliquoted and stored at -70 °C.

Each individual binding well (200 L) contained final concentrations of 1 g/mL of biotinylated EBP-Ig, 0.5 nM of [¹²⁵I]EPO (NEN Research Products, Boston, 100 Ci/ g) and 0-500 M of test compound (from a 10-50 mM stock in 100% DMSO). All wells were adjusted to a final DMSO concentration of 5%. All assay points were performed in triplicate and with each experiment a standard curve for unlabelled EPO was performed at final concentration of 2000, 62, 15, 8, 4, and 0 nM. After all additions were made, the plate was covered with an adhesive top seal and placed in the dark at room temperature overnight. The next day all liquid was aspirated from the wells to limit analyte dependent quench of the signal, and the plates were counted on a Packard TOPCOUNT Microplate Scintillation Counter. Non-specific binding (NSB) was calculated as the mean CPM of the 2000 nM EPO wells and total binding (TB) as the mean of the wells with no added unlabelled EPO. Corrected total binding (CTB) was calculated as: TB - NSB = CTB. The concentration of test compound which reduced CTB to 50% was reported as the IC₅₀. Typically the IC₅₀ value for unlabelled EPO was ca. 2-7 nM and EMP1 was 0.1 M. Table 1 lists the average % inhibition, and if determined the IC₅₀ and IC₃₀ values for compounds of Formula I, where the compound numbers refer to the compounds in the tables accompanying the preparative examples.

### Inhibition of EPO binding to EBP-Ig

**Table 1**

| cpd | % inh @ 50 M | ic30, M* | ic50, M* |
|---|---|---|---|
| 11 | 70 | nd | nd |
| 12 | 59 | nd | nd |
| 14 | 30 | nd | nd |
| 15 | 48 | nd | nd |
| 77 | 52 | 30 | 40 |
| 82 | 32 | nd | nd |
| 86 | 37 | nd | nd |
| 100 | 34 | nd | nd |
| 101 | 32 | nd | nd |
| 104 | 78 | 10 | 30 |
| 105 | 70 | 25 | 35 |
| 107 | 78 | 30 | 42 |
| 108 | 81 | 23 | 36 |
| 110 | 54 | 6 | 10 |
| 112 | 59 | 2 | 10 |
| 114 | 37 | 10 | nd |
| 115 | 35 | nd | nd |
| 116 | 32 | nd | nd |
| 117 | 34 | nd | nd |
| 118 | 36 | 2 | 10 |
| 119 | 34 | nd | nd |
| 120 | 35 | nd | nd |
| 121 | 45 | 6 | nd |
| 137 | 60 | 5 | 30 |
| 139 | 46 | 2 | 10 |
| 178 | 36 | nd | nd |
| 179 | 30 | nd | nd |
| 183 | 36 | nd | nd |
| 184 | 53 | 10 | nd |
| 203 | 37 | 50 | nd |
| 211 | 62 | 20 | 65 |
| 220 | 45 | 30 | 50 |
| 221 | 48 | 10 | 80 |
| 222 | 56 | 5 | nd |
| 224 | 51 | 25 | 50 |
| 227 | 48 | 20 | 50 |
| 230 | 42 | nd | nd |
| 231 | 36 | nd | nd |
| 235 | 49 | 20 | 50 |
| 237 | 55 | 30 | 70 |
| 238 | 39 | nd | nd |
| 239 | 46 | 8 | 50 |
| 243 | 75 | 2 | 18 |
| 244 | 66 | 1 | 28 |
| 246 | 79 | 10 | 75 |
| 247 | 47 | 7 | 18 |
| 248 | 56 | 7 | 20 |
| 249 | 72 | 7 | 10 |
| 250 | 78 | 7 | 20 |
| 251 | 49 | 10 | 45 |
| 261 | 51 | 1.5 | 2 |
| 262 | 93 | 1 | 1.5 |
| 263 | 88 | 1 | 1.5 |
| 264 | 89 | 1.5 | 8 |
| 265 | 65 | 1 | 6 |
| 266 | 82 | 1 | 4 |
| 267 | 83 | 2 | 6 |
| 268 | 40 | nd | nd |
| 269 | 55 | 8 | 85 |
| 270 | 56 | 7 | 100 |
| 271 | 77 | 2 | 7 |
| 272 | 78 | 5 | 10 |
| 285 | 41 | nd | nd |
| 286 | 46 | 35 | 65 |
| 287 | 36 | nd | nd |

| | | | |
|---|---|---|---|
| *nd = not determined | | | |

### PREPARATIVE EXAMPLES

Unless otherwise noted, materials used in the examples were obtained from commercial suppliers and were used without further purification. Melting points were determined on a Thomas Hoover apparatus and are uncorrected. Proton nuclear magnetic resonance (¹H NMR) spectra were measured in the indicated solvent with tetramethylsilane (TMS) as the internal standard using a Bruker AC-300 NMR spectrometer. NMR chemical shifts are expressed in parts per million (ppm) downfield from internal TMS using the d scale. ¹H NMR data are tabulated in order: multiplicity, (s, singlet; d, doublet; t, triplet; q, quartet; m, multiplet), number of protons, coupling constant in Hertz). Electrospray (ES) mass spectra (MS) were determined on a Hewlett Packard Series 1090 LCMS Engine. Elemental analyses were performed by Quantitative Technologies, Inc. (QTI), PO Box 470, Salem Industrial Park, Bldg #5, Whitehouse, NJ 08888-0470. Analytical thin layer chromatography (TLC) was done with Merck Silica Gel 60 F₂₅₄ plates (250 micron). Medium pressure liquid chromatography (MPLC) was done with Merck Silica Gel 60 (230-400 mesh).

### Example 1

### N,N-bis(3-Phenoxycinnamyl)Glu(O-t-Bu)-OMe and N-(3-phenoxycinnamyl)Glu(O-t-Bu)-OMe

A solution of 500 mg (1.97 mmol) of H-Glu(O-t-Bu)OMe•HCl, 997 mg (3.45 mmol) of 3-phenoxycinnamyl bromide (Jackson, W. P.; Islip, P. J.; Kneen, G.; Pugh, A.; Wates, P. J. *J.Med.Chem.* **31** *1988;* 499-500), and 0.89 mL (5.1 mmol, 660 mg) of DIEA in 5 mL of DMF was stirred under N₂ at room temperature for 40 h. The mixture was partitioned between EtOAc and water and the organic layer was washed with water and brine. After drying over Na₂SO₄, the organic solution was concentrated to give 1.24 g of orange oil. The crude residue was purified by MPLC using a solvent gradient ranging from 10-30% EtOAc/hexanes to give two products. The less polar product (235 mg, 19% based on starting amino acid), cpd 96, was isolated as a pale yellow oil; ¹H NMR (CDCl₃, 300 MHz) 1.39 (s, 9H), 2.0 (m, 2H), 2.33 (dt, 2H, J = 2, 7 Hz), 3.24 (dd, 2H, J = 8, 15 Hz), 3.5, (m, 3H), 3.69 (s, 3H), 6.13 (m, 2H), 6.47 (d, 2H, J = 16 Hz), 6.86 (dd, 2H, J = 1.5, 8 Hz), 7.0-7.4 (complex, 16H); MS (ES+) m/z 634 (MH+). The more polar product (422 mg, 50% based on starting amino acid), N-(3-phenoxycinnamyl)Glu(O-t-Bu)-OMe, was isolated as a pale yellow oil; ¹H NMR (CDCl₃, 300 MHz) 1.42 (s, 9H), 1.9 (m, 2H), 2.35 (t, 2H, J = 7.5 Hz), 3.2-3.4 (complex, 3H), 3.71 (s, 3H), 6.17 (dt, 1H, J = 16, 6 Hz), 6.46 (d, 1H, J = 16 Hz), 6.87 (dd, 1H, J = 1.5, 8 Hz), 7.01 (m, 3H), 7.10 (t, 2H, J = 7.5 Hz), 7.2-7.4 (complex, 3H); MS (ES+) m/z 426 (MH+). Anal. Calcd for C₂₅H₃₁NO₅: C, 70.57; H, 7.34; N, 3.29. Found: C, 70.29; H, 7.14; N, 3.08.

### Example 2

### N-(3-Phenoxycinnamyl)Glu-OMe

A solution of 95 mg (0.22 mmol) of N-(3-phenoxycinnamyl)Glu(O-t-Bu)-OMe in 3 mL of 50% TFA/CH₂Cl₂ was stirred for 2 h at room temperature. The mixture was concentrated and the residue was dissolve in acetic acid and freeze-dried to give 117 mg of N-(3-phenoxycinnamyl)Glu-OMe as an off-white solid; ¹H NMR (CD₃OD, 300 MHz) 2.3-2.7 (complex, 4H), 3.78 (s, 3H), 3.81 (d, 2H, J = 7 Hz), 4.09 (t, 1H, J = 5 Hz), 6.17 (dt, 1H, J = 16, 7 Hz), 6.55 (d, 1H, J = 16 Hz), 6.9 (m, 4H), 7.11 (t, 2H, J = 7.5 Hz), 7.3 (m, 4H); MS (ES+) m/z 370 (MH+), 209. Anal. Calcd for C₂₁H₂₃NO₅•C₂HF₃O₂: C, 57.14; H, 5.00; N, 2.90. Found: C, 57.07; H, 5.02; N, 2.73.

### Example 3

### N,N-bis(3-Phenoxycinnamyl)Asp(O-t-Bu)-O-t-Bu

### cpd 106

A solution of 1.00 g (3.55 mmol) of Asp(O-t-Bu)-O-t-Bu•HCl, 2.05 g (7.1 mmol) of 3-phenoxycinnamyl bromide, and 1.86 mL (10.7 mmol, 1.38 g) of DIEA in 15 mL of DMF was heated under N₂ at 60 °C overnight. Additional 3-phenoxycinnamyl bromide (1.0 g, 3.4 mmol) and DIEA (0.95 mL, 0.705 g, 5.4 mmol) were added and heating was continued for an additional 14 h. The mixture was cooled and partitioned between EtOAc and water. The organic layer was washed twice with water, once with brine, and was dried over Na₂SO₄. The solution was concentrated to give 3.5 g of an amber oil which was purified by MPLC using a solvent gradient ranging from 2.5-3% EtOAc/hexanes to afford 1.21 g of cpd **106** as a pale yellow oil; ¹H NMR (CDCl₃, 300 MHz) 1.41 (s, 9H), 1.48 (s, 9H), 2.49 (dd, 1H, J = 7.5, 15.5 Hz), 2.70 (dd, 1H, J =7.5, 15.5 Hz), 3.26 (dd, 2H, J = 7.5, 14.5 Hz), 3.47 (dd, 2H, J = 4, 14.5 Hz), 3.88 (t, 1H, J = 7.5), 6.13 (m, 2H), 6.48 (d, 2H, J = 16 Hz), 6.86 (dd, 2H, J = 2, 8 Hz), 7.0 (m, 6H), 7.1 (m, 4H), 7.2-7.4 (complex, 6H); MS (ES+) m/z 662 (MH+).

### Example 4

### N,N-bis(3-Phenoxycinnamyl)Asp-OH

### cpd 107

A solution of 1.14 g (1.62 mmol) of cpd **106** in 16 mL of 50% TFA/CH₂Cl₂ was stirred at room temperature for 24 h. The solution was concentrated and pumped to give 1.37 g (~100%) cpd 107 as an amber oil; ¹H NMR (CD₃OD, 300 MHz) 3.1 (m, 2H), 4.0 (dd, 2H, J = 8, 14 Hz), 4.27 (dd, 2H, J = 8, 16 Hz), 4.70 (t, 1H, J = 4 Hz), 6.38 (2H, dt, J = 16, 8 Hz), 6.7-7.4 (complex, 20H); MS (ES-) m/z 562 ([M-H]+).

### Example 5

### N,N-bis(4-Benzyloxybenzyl)Lys(Boc)-OMe (cpd 111) and N-(4-Benzyloxybenzyl)Lys(Boc)-OMe

A solution of 594 mg (2.0 mmol) of Lys(Boc)-OMe•HCl, 524 mg (2.25 mmol) of 4-benzyloxybenzyl chloride, 75 mg (0.5 mmol), of NaI, and 0.61 mL (3.5 mol, 452 mg) of DIEA was warmed at 50-70 °C under N₂ overnight. The mixture was cooled and partioned between EtOAc and water. The organic layer was washed twice with water, once with brine, and was dried over Na₂SO₄. The organic solution was concentrated to give 0.83 g of amber oil which was purified by MPLC using a solvent gradient ranging from 15-40% EtOAc/hexanes to give two products. The less polar product (296 mg), cpd 111, was isolated as a pale yellow oil; ¹H NMR (CDCl₃, 300 MHz) 1.28 (m, 4H), 1.43 (s, 9H), 1.70 (m, 2H), 3.03 (m, 2H), 3.28 (t, 1H, J = 7 Hz), 3.40 (d, 2H, J = 13.5 Hz), 3.74 (s, 3H), 3.81 (d, 2H, J = 13.5 Hz), 5.05 (2, 4H), 6.92 (d, 4H, J = 8.5), 7.23 (d, 4H, J = 8.5), 7.25-7.5 (complex, 10H); MS (ES+) m/z 653 (MH+). The more polar product (406 mg), N-(4-Benzyloxybenzyl)Lys(Boc)-OMe, was isolated as a white solid; ¹H NMR (CDCl₃, 300 MHz) 1.4 (, 4H), 1.43 (s, 9H), 1.65 (m, 3H), 3.08 (m, 2H), 3.23 (t, 1H, J = 6.5 Hz), 3.54 (d, 1H, J = 12.5 Hz), 3.71 (s, 3H), 3.73 (d, 1H, J = 12.5 Hz), 5.05 (s, 2H), 6.92 (d, 2H, J = 8.5 Hz), 7.23 (d, 2H, J = 8.5 Hz), 7.25-7.5 (complex, 5H); MS (ES+) m/z 457 (MH+).

### Example 6

### N-(4-Benzyloxybenzyl)-N-(3-nitrobenzyl)Lys(Boc)-OMe

### cpd 113

A solution of 374 mg (0.82 mmol) of N-(4-Benzyloxybenzyl)Lys(Boc)-OMe, 221 mg (1.03 mmol) of 4-nitrobenzyl bromide, and 197 L (1.13 mmol, 146 mg) of DIEA was warmed at 50-70 °C for 4 h, then at 40-50 °C overnight. After the addition of 0.2 mL of 1N aqueous HCl, the mixture was partioned between EtOAc and water. The organic layer was washed twice with water, once with brine, and was dried over Na₂SO₄. The organic solution was concentrated to give 610 mg of an amber oil which was purified by MPLC 1:3 EtOAc/hexanes to afford 436 mg (90%) cpd 113 as a pale yellow oil; ¹H NMR (CDCl₃, 300 MHz) 1.35 (m, 4H), 1.42 (s, 9H), 1.75 (broad q, 2H, J = 8 Hz), 3.06 (broad q, 2H, J = 6 Hz), 3.28 (t, 1H, J = 7.5 Hz), 3.48 (d, 1H, J = 13.5 Hz), 3.66 (d, 1H, J = 14.5 Hz), 3.76 (s, 3H), 3.79 (d, 1H, J = 13.5 Hz), 3.97 (d, 1H, J = 14.5 Hz), 4.47 (broad s, 1H), 5.05 (s, 2H), 6.93 (d, 2H, J = 8.5 Hz), 7.22 (d, 2H, J = 8.5 Hz), 7.3-7.5 (complex, 6H), 7.65 (d, 1H, J = 7.5 Hz), 8.09 (d, 1H, J = 8 Hz), 8.22 (s, 1H); MS (ES+) m/z 592 (MH+).

### Example 7

### N-(3-Aminobenzyl)-N-(4-benzyloxybenzyl)Lys(Boc)-OMe

A solution of 361 mg (0.61 mmol) of cpd 113 and 835 mg (3.7 mmol) of SnCl₂ dihydrate was stirred under N₂ at room temperature for 6 h. The slightly cloudy mixture was poured into 200 mL of 5% aqueous Na₂CO₃ with rapid stirring. The resulting milky suspension was extracted with three 75 mL portions of CH₂Cl₂ and the combined organic layers were washed with brine and dried over Na₂SO₄. The extracts were concentrated to give 344 mg of colorless oil which was purified by MPLC using 1:2 EtOAc/hexanes to provide 291 mg of N-(3-aminobenzyl)-N-(4-benzyloxybenzyl)Lys(Boc)-OMe as a yellow oil; ¹H NMR (CDCl₃, 300 MHz) 1.25 (m, 4H), 1.44 (s, 9H), 1.70 (m, 2H), 3.31 (dd, 1H, J = 6, 9 Hz), 3.38 (d, 1H, J =14 Hz), 3.40 (d, 1H, J =13.5 Hz), 3.74 (s, 3H), 3.81 (d, 1H, J = 14 Hz), 3.83 (d, 1H, J = 13.5 Hz), 4.52 (broad s, 1H), 5.05 (s, 2H), 6.50 (broad d, 1H, J = 8 Hz), 6.70 (m, 2H), 6.92 (d, 2H, J = 8.5 Hz), 7.08 (t, 1H, J = 7.5 Hz), 7.2-7.5 (complex, 7H); MS (ES+) m/z 562 (base, MH+), 506.

### Example 8

### N-(4-Benzyloxybenzyl)-N-(3-((2-furancarbonyl)amino)benzyl)Lys-Ome

### cpd 117

A solution of 42 mg (0.075 mmol) of N-(3-aminobenzyl)-N-(4-benzyloxybenzyl)Lys(Boc)-OMe and 12 L (12 mg, 0.15 mmol) of pyridine in 0.5 mL of 1,2-dichloroethane was combined with 8.1 L (11 mg, 0.083 mmol) and stirred under N₂ overnight. EtOAc (3 mL) was added and the solution was washed twice with 2 mL of water and 2 mL of saturated aqueous NaHCO₃. The EtOAc solution was filtered through a pad of Na₂SO₄ and concentrated to give 44 mg of N-(4-benzyloxybenzyl)-N-(3-((2-furancarbonyl)amino)benzyl)Lys(Boc)-OMe; MS (ES+) m/z 356 (MH+). The Boc-protected intermediate was stirred in 2 mL of 50% TFA/CH₂Cl₂ for 2 h and was concentrated and pumped at high vacuum to provide 66 mg of cpd 117 as the bis-TFA salt; ¹H NMR (CD₃OD, 300 MHz) 1.55 (m, 2H), 1.65 (m, 2H), 2.10 (m, 2H), 2.93 (t, 2H, J = 7 Hz), 3.68 (t, 1H, J = 7 Hz), 3.78 (s, 3H), 4.20 (m, 4H), 5.09 (s, 2H), 6.66 (dd, 1H, J = 1.5, 3.5 Hz), 7.03 (d, 2H, J = 8.5 Hz), 7.1-7.6 (complex, 11H), 7.76 (m, H), 8.07 (m, 1H); MS (ES+) m/z 556 (base, MH+), 360, 197.

### Example 9

### N,N-bis(3-Nitrobenzyl)Asp(O-t-Bu)-O-t-Bu

### cpd 62

A solution of 0.50 mg (1.77 mmol) of Asp(O-t-Bu)-O-t-Bu•HCl, 1.17 g (5.42 mmol) of 3-nitrobenzyl bromide, and 1.25 mL (0.93 g, 7.2 mmol) of DIEA in 6 mL of DMF was stirred at room temperature under N₂ for 24 h and was heated at 70-80 °C overnight. The reaction mixture was partitioned between EtOAc and water and the organic layer was washed twice with water and once with brine. After drying over Na₂SO₄, the organic solution was concentrated to give 0.86 g of a yellow oil which was purified by MPLC using 1:9 EtOAc/hexanes to afford 0.849 g (93%) cpd 62 as a pale yellow oil; ¹H NMR (CDCl₃, 300 MHz) 1.43 (s, 9H), 1.57 (s, 9H), 2.59 (dd, 1H, J = 7.5, 16 Hz), 2.76 (dd, 1H, J = 7, 16 Hz), 3.72 (t, 1H, J = 7.5 Hz), 3.78 (d, 2H, J = 14 Hz), 3.92 (d, 2H, J = 14 Hz), 7.47 (t, 2H, J = 8 Hz), 7.67 (d, 2H, J = 7.5 Hz), 8.09 (broad d, 2H J = 8 Hz), 8.16 (broad s, 2H); MS (ES+) m/z 538 (MNa+), 516 (base, MH+), 460, 404, 237.

### Example 10

### N,N-bis(3-Aminobenzyl)Asp(O-t-Bu)-O-t-Bu

A solution of 0.644 g (1.25 mmol) of cpd 62 and 2.82 g (12.5 mmol) of SnCl₂•2 H₂O in 12 mL of absolute EtOH was refluxed for 1.5 h. The mixture was cooled and poured into 300 mL of 5% aqueous Na₂CO₃ with rapid stirring. The cloudy mixture was extracted with three 150 mL portions of CH₂Cl₂ and the organic extracts were washed with brine and dried over Na₂SO₄. The CH₂Cl₂ solution was concentrated to afford 210 mg (37%) of N,N-bis(3-aminobenzyl)Asp(O-t-Bu)-O-t-Bu as a cloudy yellow oil which was used without purification; ¹H NMR (CDCl₃, 300 MHz) 1.40 (s, 9H), 1.52 (s, 9H), 2.48 (dd, 1H, J = 7, 16 Hz), 2.76 (dd, 1H, J = 8, 16 Hz), 3.48 (d, 2H, J = 14 Hz), 3.55 (m, 1H), 3.73 (d, 2H, J = 14 Hz), 6.56 (broad d, 2H J = 8 Hz), 6.70 (broad s, 2H), 6.77 (d, 2H, J = 7.5 Hz), 7.08 (t, 2H, J = 8 Hz); MS (ES+) m/z 478 (MNa+), 456 (base, MH+), 400, 344.

### Example 11

### N,N-bis(3-(4-Methylbenzoyl)aminobenzyl)Asp(O-t-Bu)-O-t-Bu

To a solution of 109 mg (0.24 mmol) of N,N-bis(3-aminobenzyl)Asp(O-t-Bu)-O-t-Bu, 29 mg (0.24 mmol) ofDMAP, 125 L (93 mg, 0.72 mmol) of DIEA in 1 mL of CH₂Cl₂ was added 95 L (111 mg, 0.72 mmol) of 4-methylbenzoyl chloride. The solution was stirred under N₂ overnight and was then partitioned between EtOAc and water. The organic layer was washed with saturated aqueous NaHCO₃ and brine, dried over Na₂SO₄, and concentrated to give 177 mg of yellow oil. The crude material was purified by MPLC using a solvent gradient ranging from 20-25% EtOAc/hexanes to provide 87 mg of N,N-bis(3-(4-methylbenzoyl)aminobenzyl)Asp(O-t-Bu)-O-t-Bu as a pale yellow oil; ¹H NMR (CDCl₃, 300 MHz) 1.36 (s, 9H), 1.55 (s, 9H), 2.35 (s, 6H), 2.53 (dd, 1H, J = 6, 16 Hz), 2.76 (dd, 1H, J = 9, 16 Hz), 3.69 (d, 2H, J = 14), 3.77 (dd, 1H, J = 6, 9 Hz), 3.83 (d, 2H, J = 14), 7.01 (m, 6H), 7.26 (t, 2H, J = 8 Hz), 7.59 (m, 6H), 8.11 (s, 2H), 8.49 (s, 2H); MS (ES+) m/z 714 (MNa+), 692 (base, MH+), 636, 580.

### Example 12

### N,N-bis(3-(4-Methylbenzoyl)aminobenzyl)Asp-OH

### cpd 64

A solution of 87 mg (0.13 mmol) of N,N-bis(3-(4-methylbenzoyl)aminobenzyl)Asp(O-t-Bu)-O-t-Bu in 1 mL of 50% TFA/CH₂Cl₂ was stirred overnight. The mixture was concentrated and the residue was dissolved in HOAc and freeze-dried to afford 77 mg cpd 64 as a white solid; ¹H NMR (CD₃OD, 300 MHz) 2.40 (s, 6H), 2.85 (dd, 1H, J = 6, 16.5 Hz), 2.98 (dd, 1H, J = 8, 16.5 Hz), 4.02 (d, 2H, J = 13.5 Hz), 4.08 (d, 4H, J = 13.5 Hz), 4.10 (t, 1H, J = 6.5 Hz), 7.22 (m, 6H), 7.34 (t, 2H, J = 7.5 Hz), 7.60 (broad d, 2H, J = 9 Hz), 7.76 (d, 4H, J = 8 Hz), 7.88 (broad s, 2H); MS (ES+) m/z 580 (base, MH+).

### Example 13

### [N-Cbz-Glu(O-t-Bu)-NHCH₂CH₂OCH₂]₂

To a solution of 1.69 g (5.0 mmol) of N-Cbz-Glu(O-t-Bu)-OH, 0.365 mL (0.371 g, 2.5 mmol) of 1,8-diamino-3,6-dioxaoctane, 0.743 g (5.5 mmol) of HOBT, and 1.05 mL (0.776 g, 6.0 mmol) of DIEA in 15 mL of CH₂Cl₂ was added 1.05 g (5.5 mmol) of EDCI in one portion. After stirring at room temperature under N₂ overnight, the mixture was partitioned between EtOAc and 10% aqueous citric acid. The organic layer was washed with water, saturated NaHCO₃, and brine, dried over Na₂SO₄, and concentrated to give 1.87 g of (N-Cbz-Glu(O-t-Bu)-NHCH₂CH₂OCH₂)₂ as a colorless oil; ¹H NMR (CD₃OD, 300 MHz) 1.43 (s, 18H), 1.85 (m, 2H), 2.05 (m, 2H), 2.31 (t, 4H, J = 8 Hz), 3.37 (t, 4H, J = 5 Hz), 3.52 (t, 4H, J = 5 Hz), 3.58 (s, 4H), 4.15 (m, 2H), 5.09 (dd, 4H, J = 12, 16 Hz), 7.30 (m, 10H); MS (ES+) m/z 809 (base, MNa+), 787 (MH+).

### Example 14

### [Glu(O-t-Bu)-NHCH₂CH₂OCH₂]₂

Ammonium formate (0.78 g, 12.4 mmol) and 0.16 g of 10% palladium on carbon were added to a solution of (N-Cbz-Glu(O-t-Bu)-NHCH₂CH₂OCH₂)₂ in 12 mL of MeOH and the resulting suspension was stirred under N₂ at room temperature overnight. The mixture was diluted with CH₂Cl₂ and filtered through a Celite pad. The solids were washed thoroughly with CH₂Cl₂ and the combined organic filtrates were concentrated to dryness. The residue was partitioned between CH₂Cl₂ and saturated aqueous NaHC0₃, washed with brine, dried over Na₂SO₄, and concentrated to give 1.13 g of (Glu(O-t-Bu)-NHCH₂CH₂OCH₂)₂ as a colorless oil; 1.44 (s, 18H), 1.81 (m, 2H), 2.08 (m, 2H), 2.35 (m, 4H), 3.39 (dd, 2H, J = 5, 7.5 Hz), 3.47 (t, 4H, J = 5 Hz), 3.58 (t, 4H, J = 5 Hz), 7.53 (m, 2H).

### Example 15

### [N,N-bis(4-Benzyloxybenzyl)Glu(O-t-Bu)-NHCH₂CH₂OCH₂]₂

### cpd 245

A solution of 199 mg (0.384 mmol) of [Glu(O-t-Bu)-NHCH₂CH₂OCH₂]₂, 403 mg (1.73 mmol) of 4-benzyloxybenzyl chloride, 30 mg (0.2 mmol) of NaI, and 334 L (248 mg, 1.92 mmol) of DIEA was stirred under N₂ at room temperature for several days. The solution was partitioned between EtOAc and water and the organic layer was washed three times with water and once with brine. After drying over Na₂SO₄, the solution was concentrated to give 528 mg of yellow oil which was purified by MPLC using a solvent gradient ranging from 42-50% EtOAc/hexanes to afford 318 mg (64%) of cpd 245 as a white foam; ¹H NMR (CDCl₃, 300 MHz) 1.42 (s, 18H), 2.01 (m, 4H), 2.38 (m, 2H), 2.55 (m, 2H), 3.03 (dd, 2H, J = 5, 8 Hz), 3.31 (m, 2H), 3.4-3.6 (complex, 18H), 4.99 (s, 8H), 6.89 (d, 8H, J = 8.5), 7.1-7.4 (complex, 30H).

### Example 16

### [N,N-bis(4-Benzyloxybenzyl)GluNHCH₂CH₂OCH₂]₂

### cpd 246

A solution of 219 mg (0.168 mmol) of cpd **245** in 2 mL of 33% TFA/CH₂Cl₂ was stirred ad room temperature overnight. The mixture was concentrated to give a crude product which was dissolved in HOAc and freeze-dried to afford 251 mg of cpd 246 as an amber oil; ¹H NMR (CD₃OD, 300 MHz) 2.1-2.6 (complex, 8H), 3.3-3.6 (complex, 8H), 3.57 (s, 4H), 3.78 (m, 2H), 4.25 (broad d, 4H, J = 14 Hz), 4.36 (broad d, 4H, J = 14 Hz), 5.09 (s, 8H), 7.03 (d, 8H, J = 8 Hz), 7.3-7.5 (complex, 28H); MS (ES+) m/z 1192 (MH+), 995, 596, 197 (base).

### Example 17

### [N-(3-Phenoxybenzyl)Glu(O-t-Bu)-NHCH₂CH₂OCH₂]₂

A solution of 680 mg (0.76 mmol) of [Glu(O-t-Bu)-NHCH₂CH₂OCH₂]₂ and 278 L (317 mg, 1.6 mmol) of 3-phenoxybenzaldehyde in 3 mL of TMOF was stirred overnight at room temperature under N₂. The mixture was concentrated and pumped at high vacuum to give a colorless oil which was dissolved in 3 mL of CH₂Cl₂ and treated with 678 mg (3.2 mmol) of NaBH(OAc)₃. After stirring under N₂ for 2 days, 50 mL of saturated aqueous NaHCO₃ was added and the mixture was extracted with CH₂Cl₂. The organic layers were combined, dried over Na₂SO₄, and concentrated and the crude product (1.01 g) was purified by MPLC using a solvent gradient ranging from 2-4% MeOH/CH₂Cl₂ to afford 490 mg of [N-(3-phenoxybenzyl)Glu(O-t-Bu)-NHCH₂CH₂OCH₂]₂ as a colorless oil; ¹H NMR (CDCl₃, 300 MHz) 1.41 (s, 18H), 1.89 (m, 4H), 2.31 (m, 4H), 3.12 (t, 2H, J = 6 Hz), 3.45 (m, 8H), 3.55 (s, 4H), 3.60 (d, 2H, J = 13.5 Hz), 3.73 (d, 2H, J = 13.5 Hz), 6.86 (dd, 2H, J = 1.5, 8 Hz), 7.00 (m, 8H), 7.2-7.4 (complex, 8H); MS (ES+) m/z 883 (MH+), 589,442,414,386 (base), 183.

### Example 18

### [N-(3-Nitrobenzyl)-N-(3-phenoxybenzyl)-Glu(O-t-Bu)-NHCH₂CH₂OCH₂]₂

DIEA (269 L, 199 mg, 1.54 mmol), 3-nitrobenzyl bromide (322 mg, 1.49 mmol), and [N-(3-phenoxybenzyl)Glu(O-t-Bu)-NHCH₂CH₂OCH₂]₂ (482 mg, 0.546 mmol) were combined in 2 mL of DMF and heated at 60-70 °C under N₂ for 2 days. The reaction mixture was cooled and partitioned between 100 mL of EtOAc and water. The organic layer was washed with three times with water and once with brine, dried over Na₂SO₄, and concentrated to give 661 mg (~100%) of [N-(3-nitrobenzyl)-N-(3-phenoxybenzyl)-Glu(O-t-Bu)-NHCH₂CH₂OCH₂]₂ which was used without purification; MS (ES+) m/z 1154 (MH+), 577, 130 (base).

### Example 19

### [N-(3-Aminobenzyl)-N-(3-phenoxybenzyl)-Glu(O-t-Bu)-NHCH₂CH₂OCH₂]₂

A solution of 661 mg (0.54 mmol) of crude [N-(3-nitrobenzyl)-N-(3-phenoxy-benzyl)-Glu(O-t-Bu)-NHCH₂CH₂OCH₂]₂ and 2.71 g (12.0 mmol) of SnCl₂ • 2 H₂O in 20 mL of absolute EtOH was refluxed under N₂ for 30 min. The cooled solution was poured into 500 mL of 2.5% aqueous Na₂CO₃ with rapid stirring and the resulting cloudy mixture was extracted thoroughly with EtOAc. The slightly cloudy organic extracts were washed twice with brine, dried over Na₂SO₄, anc concentrated to give 604 mg of yellow oil which was purified by MPLC using 3% MeOH/CH₂Cl₂ to provide 350 mg (59%) of [N-(3-aminobenzyl)-N-(3-phenoxybenzyl)-Glu(O-t-Bu)-NHCH₂CH₂OCH₂]₂ as a pale yellow oil; ¹H NMR (CDCl₃, 300 MHz) 1.41 (s, 18H), 1.97 (m, 4H), 2.25 (m, 4H), 2.48 (m, 4H), 3.03 (dd, 2H, J = 5, 8 Hz), 3.30 (m, 2H), 3.4-3.8 (complex, 24H), 6.47 (d, 2H, J = 7.5 Hz), 6.65 (m, 4H), 6.85 (d, 2H, J = 9.5 Hz), 6.9-7.15 (complex, 12H), 7.2-7.4 (complex, 8H); MS (ES+) m/z 1094 (MH+), 547 (base).

### Example 20

### [N-(3-Phenoxybenzyl)-N-(3-(pentanoylamino)benzyl)-Glu-NHCH₂CH₂OCH₂]₂

### cpd 247

Pentanoyl chloride (16 uL, 16 mg, 0.136 mmol) was added dropwise to a solution of 68 mg (0.062 mmol) of [N-(3-aminobenzyl)-N-(3-phenoxybenzyl)-Glu(O-t-Bu)-NHCH₂CH₂OCH₂]₂, 20 L (20 mg, 0.25 mmol) of pyridine in 0.3 mL of 1,2-dichloroethane. The mixture was shaken under N₂ overnight and was then partitioned between EtOAc and water. The organic layer was washed with saturated aqueous NaHCO₃, dried over Na₂SO₄, and concentrated to give 77 mg of [N-(3-phenoxybenzyl)-N-(3-(pentanoylamino)benzyl)-Glu(O-t-Bu)-NHCH₂CH₂OCH₂]₂; MS (ES+) m/z 1073, 575 (base, MH+/2). The crude product was dissolved in 1 mL of 50% TFA/CH₂Cl₂ and allows to stand overnight. The solution was concentrated and the resulting oil was dissolved in HOAc and freeze-dried to provide 82 mg of cpd 247; ¹H NMR (CD₃OD, 300 MHz) 3.98 (t, 6H, J = 7.5 Hz), 1.39 (sextet, 4H, J = 7.5 Hz), 1.66 (quintet, 4H, J = 7.5 Hz), 1.65 (m, 2H), 1.78 (m, 2H), 2.35 (t, 4H, J = 7.5 Hz), 2.45 (m, 4H), 3.38 (m, 4H), 3.50 (t, 2H, J = 5), 3.57 (m, 4H), 4.10 (broad s, 8H), 6.9-7.25 (complex, 14H), 7.25-7.4 (complex, 10H), 7.71 (s, 2H); MS (ES+) m/z 1150 (MH+), 575 (base).

### Example 21

### [N-Cbz-Lys(Boc)-NHCH₂CH₂]₃N

A solution of 1.0 g (2.63 mmol) of N-Cbz-Lys(Boc)OH, 0.131 mL (0.128 g, 0.876 mmol) of tris(2-aminoethyl)amine, 0.391 g (2.98 mmol) of HOBt, 0.555 g (2.89 mmol) of EDCI, and 0.55 mL (0.408 g, 3.16 mmol) of DIEA in 5 mL of CH₂Cl₂ was stirred under N₂ at room temperature overnight. The mixture was diluted with EtOAc and washed with 10% aqueous citric acid, saturated aqueous NaHCO₃, and brine. The solution was dried over Na₂SO₄ and concentrated to give 0.872 g of [N-Cbz-Lys(Boc)-NHCH₂CH₂]₃N as an off-white solid; ¹H NMR (CD₃OD, 300 MHz) 135 (m, 12H), 1.40 (s, 27H), 1.60 (m, 3H), 1.72 (m, 3H), 2.51 (m, 6H), 2.99 (m, 6H), 3.10 (m, 3H), 3.21 (m, 3H), 4.12 (m, 3H), 5.00 (d, 3H, J = 12.5 Hz), 5.08 (d, 3H, J = 12.5 Hz), 7.29 (m, 15H); MS (ES+) m/z 1243 (base, MH+), 567, 467.

### Example 22

### [Lys(Boc)-NHCH₂CH₂]₃N

A solution of 0.841 g (0.682 mmol) [N-Cbz-Lys(Boc)-NHCH₂CH₂]₃N, 0.252 g of 10% Pd-C, and 0.774 g (12.3 mmol) of ammonium formate in 10 mL of MeOH was stirred for 5 h at room temperature under N₂. The mixture was filtered through a Celite pad, the solids were washed with CH₂Cl₂, and the reslulting solution was concentrated to dryness. The residue was partitioned between CH₂Cl₂ and brine; the organic layer was dried over Na₂SO₄ and concentrated to provide 0.191 g of [Lys(Boc)-NHCH₂CH₂]₃N as an off-white solid; ¹H NMR (CD₃OD, 300 MHz) 1.40 (s, 27H), 1.45 (m, 12H), 1.75 (m, 6H), 2.62 (m, 6H), 3.01 (m, 6H), 3.28 (m, 6H), 3.64 (m, 3H); MS (ES+) m/z 853 (MNa+), 831 (MH+), 266 (base).

### Example 23

### [N,N-bis(3-Phenoxybenzyl)Lys(Boc)-NHCH₂CH₂]₃N

A solution of 65 mg (0.078 mmol) of [Lys(Boc)-NHCH₂CH₂]₃N, 120 L (140 mg, 0.70 mmol) of 3-phenoxybenzaldehyde, and 71 L (65 mg, 0.70 mmol) of borane-pyridine complexin 3 mL of absolute EtOH was stirred for 4 days at room temperature under N₂. The mixture was concentrated to dryness and partitioned between water and CH₂Cl₂. The organic layer was concentrated to give a yellow oil which was purified by MPLC using 2.5% MeOH/CH₂Cl₂ to give 78 mg of [N,N-bis(3-phenoxybenzyl)Lys(Boc)-NHCH₂CH₂]₃N as a yellow oil; MS (ES+) m/z 872 (base, [M-Cl₁₃H₁₂O)/2]+), 611, 443.

### Example 24

### [N,N-bis(3-Phenoxybenzyl)Lys-NHCH₂CH₂]₃N

### cpd 277

A solution of 78 mg (0.048 mmol) of [N,N-bis(3-phenoxybenzyl)Lys(Boc)-NHCH₂CH₂]₃N in 2 mL of 50% TFA/CH₂Cl₂ was stirred for 2 h at room temperature. The mixture was diluted with CH₂Cl₂, washed with water and 5% Na₂CO₃, and concentrated to give 57 mg of cpd 277 as an off-white foam; ¹H NMR (CD₃OD, 300 MHz) 1.35 (m, 6H), 1.52 (m, 6H), 1.76 (m, 6H), 2.75 (m, 6H), 3.19 (m, 6H), 3.40 (m, 6H), 3.60 (m, 9H), 3.77 (m, 6H), 6.79 (d, 6H, J = 8 Hz), 6.93 (m, 24H), 7.05 (m, 6H), 7.19 (m, 6H), 7.29 (m, 12H); MS (ES+) m/z 813 ([MH₂/2]+), 721, 542 (base, [MH/3]+).

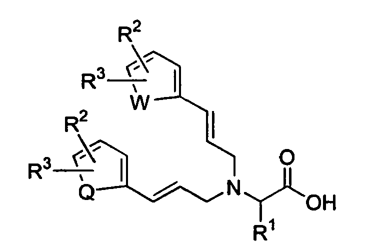

**Table 2**

| cpd | % inh | R¹ (amino acid side chain) | R² | R³ | W, Q |
|---|---|---|---|---|---|
| 11 | 70 | Asn, Asp, Gln, Glu | 3-PhO | | CH=CH |
| 12 | 59 | Cys, Met, Ser, Thr | 3-PhO | | CH=CH |
| 13 | nd | Arg, Gly, His, Pro | 3-PhO | | CH=CH |
| 14 | 30 | Lys(2-Cl-Cbz), Phe, Trp, Tyr | 3-PhO | | CH=CH |
| 15 | 48 | Ala, Ile, Leu, Val | 3-PhO | | CH=CH |
| 16 | nd | Glu, Asp | 2,3-benzo | | CH=CH |
| 17 | nd | Cys, Met | 2,3-benzo | | CH=CH |
| 18 | nd | Ser, Thr | 2,3-benzo | | CH=CH |
| 19 | nd | His, Arg(Mtr) | 2,3-benzo | | CH=CH |
| 20 | nd | Pro, Gly | 2,3-benzo | | CH=CH |
| 21 | nd | Phe, Tyr | 2,3-benzo | | CH=CH |
| 22 | nd | Trp, Lys(2-Cl-Cbz) | 2,3-benzo | | CH=CH |
| 23 | nd | Ile, Ala | 2,3-benzo | | CH=H |
| 24 | nd | Val,Leu | 2,3-benzo | | CH=CH |
| 25 | nd | Asn, Lys | 2,3-benzo | | CH=H |
| 26 | nd | Ala, Ile | | 3,4-benzo | CH=CH |
| 27 | nd | Arg(Mtr), Lys(2-Cl-Cbz) | | 3,4-benzo | CH=CH |
| 28 | nd | Asp, Glu | | 3,4-benzo | CH=CH |
| 29 | nd | Cys, Met | | 3,4-benzo | CH=H |
| 30 | nd | Gly, Pro | | 3,4-benzo | CH=CH |
| 31 | nd | His, Lys | | 3,4-benzo | CH=CH |
| 32 | nd | Leu, Val | | 3,4-benzo | CH=CH |
| 33 | nd | Lys(2-Cl-Cbz), Phe | | 3,4-benzo | CH=CH |
| 34 | nd | Ser, Thr | | 3,4-benzo | CH=CH |
| 35 | nd | Trp, Tyr | | 3,4-benzo | CH=CH |

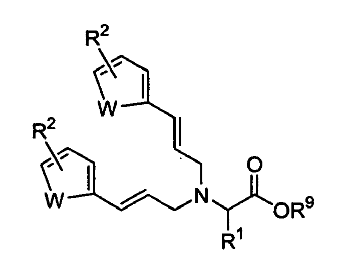

**Table 3**

| cpd | EPO / EBP-Ig %inh @ 50 M | R¹ | R² | R⁹ | W, Q | MS MH+ |
|---|---|---|---|---|---|---|
| 36 | nd | CH₃ | 4-CF₃ | H | CH=CH | 458 |
| 37 | 19 | H | 4-CF₃ | H | CH=CH | 430 |
| 38 | nd | (CH₂)₄NH(2-Cl-Cbz) | 4-F | H | CH=CH | 448 |
| 40 | nd | CH₃ | 4-F | H | CH=CH | 223 |
| 41 | nd | CH₂CO₂H | 4-F | H | CH=CH | 266 |
| 42 | nd | CH₂CH₂CO₂H | 4-F | H | CH=CH | 281 |
| 43 | nd | (CH₂)₃NHC(=NH)NH₂ | 4-F | H | CH=CH | 308 |
| 45 | nd | PhCH₂ | 4-F | H | CH=CH | 299 |
| 46 | nd | 4-HO-PhCH₂ | 4-F | H | CH=CH | 315 |
| 47 | nd | CH₂OH | 4-F | H | CH=CH | 238 |
| 48 | nd | CH(OH)CH₃ | 4-F | H | CH=CH | 253 |
| 49 | 1 | (CH₂)₃NHC(=NH)NH₂ | H | H | S | 419 |
| 50 | -6 | (CH₂)₄NH₂ | H | H | S | 391 |
| 51 | nd | CH(CH₃)CH₂CH₃ | H | H | S | 376 |
| 52 | 21 | CH₂CH₂CO₂H | H | H | S | 392 |
| 53 | 14 | CH₂CO₂H | H | H | S | 378 |
| 54 | 18 | CH₃ | H | H | S | 334 |
| 55 | 4 | CH₂CH₂CONH₂ | H | H | S | 391 |
| 56 | nd | (CH₂)₄NHCbz | H | Me | S | 539 |
| 57 | 0 | (CH₂)₄NHCbz | H | CH₂Ph | S | 615 |
| 58 | nd | CH₂(indol-3-yl) | H | Me | S | 463 |
| 59 | 26 | CH₂CH₂CO₂-t-Bu | H | Me | S | 462 |
| 60 | 9 | CH₂CH₂CO₂Et | H | Me | S | 434 |
| 61 | 14 | CH₂CH₂CO₂H | H | Me | S | 406 |

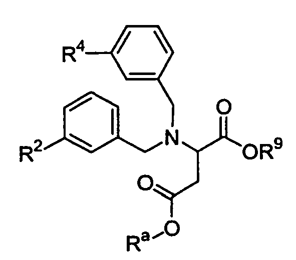

**Table 4**

| cpd | EPO/EBP-Ig %inh @ 50 M | R^{a} | R² | R⁴ | R⁹ | MS MH+ |
|---|---|---|---|---|---|---|
| 62 | nd | t-Bu | NO₂ | NO₂ | t-Bu | 516 |
| 63 | 20 | H | PhCH₂NH | PhO | H | 511 |
| 64 | -4 | H | 4-MePhCONH | 4-MePhCONH | H | 580 |
| 65 | -7 | H | 4-MePhSO₂NH | 4-MePhSO₂NH | H | 652 |
| 66 | -16 | H | 3-ClPhCH₂NH | PhO | H | 546 |
| 67 | -8 | H | 3-BrPhCH₂NH | PhO | H | 590 |
| 68 | -13 | H | 2-FPhCH₂NH | PhO | H | 529 |
| 69 | -13 | H | 2-MePhCH₂NH | PhO | H | 525 |
| 70 | -8 | H | 4-FPhCH₂NH | PhO | H | 529 |
| 71 | -6 | H | 3-ClPhCH₂NH | 4-Me-PhO | H | 560 |
| 72 | -14 | H | F₅-PhCH₂NH | 4-Me-PhO | H | 615 |
| 73 | -13 | H | 2-FPhCH₂NH | 4-Me-PhO | H | 543 |
| 74 | -7 | H | 3-CNPhCH₂NH | 4-Me-PhO | H | 550 |
| 75 | -2 | H | PhCH₂NH | 4-Me-PhO | H | 525 |

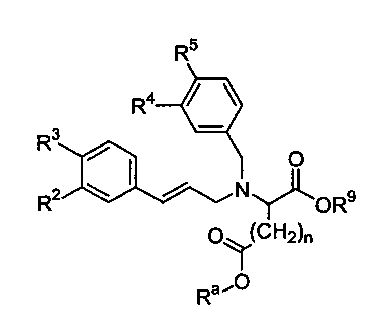

**Table 5**

| cpd | EPO / EBP-Ig %inh @ 50 M | R^{a} | R² | R³ | R⁴ | R⁵ | R⁹ | n | MS MH+ |
|---|---|---|---|---|---|---|---|---|---|
| 76 | 25 | t-Bu | PhO | H | PhO | H | t-Bu | 1 | 636 |
| 77 | 52 | H | PhO | H | PhO | H | H | 1 | 524 |
| 78 | nd | H | H | 4-MePhCONH | H | PhCH₂ O | H | 2 | 593 |
| 79 | nd | H | H | n-BuCONH | H | PhCH₂ O | H | 2 | 559 |
| 80 | nd | H | H | 2-naphthyl CONH | H | PhCH₂ O | H | 2 | 629 |
| 81 | nd | H | H | 2-furyl CONH | H | PhCH₂ O | H | 2 | 569 |
| 82 | 32 | H | H | 4-MeO-PhCONH | H | PhCH₂ O | H | 2 | 609 |
| 83 | 18 | H | H | HO₂C(CH₂)₃CONH | H | PhCH₂ O | H | 2 | 589 |
| 84 | 14 | H | H | C₂F₅CONH | H | PhCH₂ O | H | 2 | 621 |
| 85 | 20 | H | H | CF₃CONH | H | PhCH₂ O | H | 2 | 571 |
| 86 | 37 | H | H | 4-pyridyl-CONH | H | PhCH₂ O | H | 2 | 580 |
| 87 | 23 | H | H | 4-MePhSO₂NH | H | PhCH₂ O | H | 2 | 629 |
| 88 | 10.3 | H | H | HO₂CCH₂(1,1-cyclopentyl) CH₂CONH | H | PhCH₂ O | H | 2 | 643 |
| 89 | 22 | H | H | PhOCONH | H | PhCH₂ O | H | 2 | 595 |
| 90 | 29 | H | H | 4-Ph-PhCONH | H | PhCH₂ O | H | 2 | 655 |
| 91 | 19 | H | H | 4-NO₂-PhCONH | H | PhCH₂ O | H | 2 | 624 |

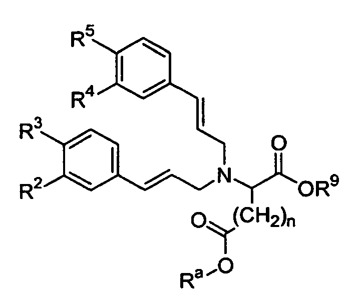

**Table 6**

| cpd | EPO / EBP-Ig %inh @ 50 M | R^{a} | R² | R³ | R⁴ | R⁵ | R⁶ | R⁹ | MS MH + |
|---|---|---|---|---|---|---|---|---|---|
| 92 | 20 | H | H | H | H | H | 2 | Me | 394 |
| 93 | 20 | t-Bu | H | H | H | H | 2 | Me | 450 |
| 94 | 25 | Et | H | H | H | H | 2 | Me | 422 |
| 95 | 15 | t-Bu | 2,3-benzo | | 2,3-benzo | | 2 | Me | 550 |
| 96 | -5 | t-Bu | PhO | H | PhO | H | 2 | Me | 634 |
| 97 | 14 | t-Bu | 3,4-benzo | | 3,4-benzo | | 2 | H | 536 |
| 98 | 12 | t-Bu | H | Ph | H | Ph | 2 | Me | 602 |
| 99 | 13 | t-Bu | 3,4-di-Cl-PhO | H | 3,4-di-Cl-PhO | H | 2 | Me | 772 |
| 100 | 34 | H | H | Ph | H | Ph | 2 | Me | 546 |
| 101 | 32 | H | 3,4-di-Cl-PhO | H | 3,4-di-Cl-PhO | H | 2 | Me | 716 |
| 102 | 5 | t-Bu | 4-t-Bu-PhO | H | 4-t-Bu-PhO | H | 2 | t-Bu | 789 |
| 103 | 17 | t-Bu | 3-CF3-PhO | H | 3-CF3-PhO | H | 2 | t-Bu | 812 |
| 104 | 78 | H | 4-t-Bu-PhO | H | 4-t-Bu-PhO | H | 2 | H | 676 |
| 105 | 70 | H | 3-CF3-PhO | H | 3-CF3-PhO | H | 2 | H | 700 |
| 106 | 20 | t-Bu | PhO | H | PhO | H | 1 | t- Bu | 662 |
| 107 | 78 | H | PhO | H | PhO | H | 2 | H | 562* |
| 108 | 81 | H | PhO | H | PhO | H | 1 | H | 550 |

| | | | | | | | | | |
|---|---|---|---|---|---|---|---|---|---|
| *[M-H]⁻ | | | | | | | | | |

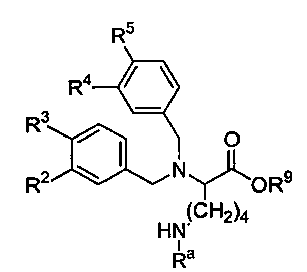

**Table 7**

| cpd | EPO / EBP-Ig %inh @ 50 M | R | R | R³ | R⁴ | R⁵ | R⁹ | MS MH + |
|---|---|---|---|---|---|---|---|---|
| 109 | 7 | Boc | PhCH₂O | H | PhCH₂O | H | Me | 653 |
| 110 | 54 | H | H | PhCH₂ O | H | PhCH₂ O | Me | 553 |
| 111 | 5 | Boc | H | PhCH₂ O | H | PhCH₂ O | Me | 653 |
| 112 | 59 | H | PhCH₂O | H | PhCH₂O | H | Me | 553 |
| 113 | 24 | Boc | H | PhCH₂ O | NO₂ | H | Me | 592 |
| 114 | 37 | H | H | PhCH₂ O | NO₂ | H | Me | 492 |
| 115 | 35 | H | H | PhCH₂ O | NH₂ | H | Me | 462 |
| 116 | 32 | H | H | PhCH₂ O | n-BuCONH | H | Me | 546 |
| 117 | 34 | H | H | PhCH₂ O | 2-furylCONH | H | Me | 556 |
| 118 | 36 | H | H | PhCH₂ O | 4-MePhCONH | H | Me | 580 |
| 119 | 34 | H | H | PhCH₂ O | i-Pr-CONH | H | Me | 532 |
| 120 | 35 | H | H | PhCH₂ O | 4-pyridyl-CONH | H | Me | 567 |
| 121 | 45 | H | H | PhCH₂ O | 2-naphthyl-CONH | H | Me | 616 |
| 122 | nd | Boc | PhCH₂NH | H | PhCH₂NH | H | Me | 651 |
| 123 | nd | Boc | 2-MePhCH₂NH | H | 2-MePhCH₂NH | H | Me | 679 |
| 124 | nd | Boc | 4-MeO-PhCH₂NH | H | 4-MeO-PhCH₂NH | H | Me | 711 |
| 125 | nd | Boc | 3,4-di-MeO-PhCH₂NH | H | 3,4-di-MeO-PhCH₂NH | H | Me | 771 |
| 126 | nd | Boc | -NH₂ | H | -NH₂ | H | Me | 471 |
| 127 | nd | H | PhCH₂NH | H | PhCH₂NH | H | Me | 551 |
| 128 | nd | H | 2-MePhCH₂NH | H | 2-MePhCH₂NH | H | Me | 579 |
| 129 | nd | H | 4-MeO-PhCH₂NH | H | 4-MeO-PhCH₂NH | H | Me | 611 |
| 130 | nd | H | 3,4-di-MeO-PhCH₂NH | H | 3,4-di-MeO-PhCH₂NH | H | Me | 671 |
| 131 | nd | H | PhCH₂CH₂N H | H | PhCH₂CH₂NH | H | Me | 579 |
| 132 | nd | HO₂CCH₂CH₂ CO | PhCH₂NH | H | PhCH₂NH | H | Me | 651 |
| 133 | nd | HO₂CCH₂CH₂ CO | 2-MePhCH₂NH | H | 2-MePhCH₂NH | H | Me | 679 |
| 134 | nd | HO₂CCH₂CH₂ CO | 4-MeO-PhCH₂NH | H | 4-MeO-PhCH₂NH | H | Me | 711 |
| 135 | nd | HO₂CCH₂CH₂ CO | 3,4-di-MeO-PhCH₂NH | H | 3,4-di-MeO-PhCH₂NH | H | Me | 771 |
| 136 | nd | HO₂CCH₂CH₂ CO | PhCH₂CH₂N H | H | PhCH₂CH₂NH | H | Me | 679 |

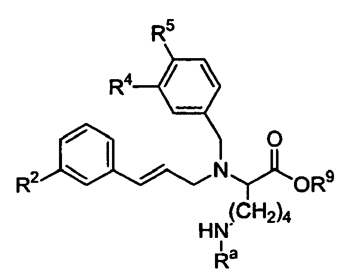

**Table 8**

| cpd | EPO / EBP-Ig %inh @ 50 M | R^{a} | R² | R⁴ | R⁵ | R⁹ | MS MH+ |
|---|---|---|---|---|---|---|---|
| 137 | nd | H | PhO | PhO | H | Me | 551 |
| 138 | nd | Boc | 4-t-Bu-PhO | PhCH₂O | H | Me | 721 |
| 139 | nd | H | 4-t-Bu-PhO | PhCH₂O | H | Me | 621 |
| 140 | nd | H | (CF₃CO)₂N | PhCH₂O | H | H | 666 |
| 141 | nd | H | PhCONH | PhCH₂O | H | H | 578 |
| 142 | nd | H | 4-pyridyl-CONH | PhCH₂O | H | H | 579 |
| 143 | nd | H | (CF₃CO)₂N | PhO | H | H | 652 |
| 144 | nd | H | PhCONH | PhO | H | H | 564 |
| 145 | nd | H | 4-pyridyl-CONH | PhO | H | H | 565 |
| 146 | nd | H | (CF₃CO)₂N | MeO | MeO | H | 620 |
| 147 | nd | H | PhCONH | MeO | MeO | H | 532 |
| 148 | nd | H | 4-pyridyl-CONH | MeO | MeO | H | 533 |
| 149 | nd | H | (CF₃CO)₂N | H | PhO | H | 652 |
| 150 | nd | H | PhCONH | H | PhO | H | 564 |
| 151 | nd | H | 4-pyridyl-CONH | H | PhO | H | 565 |
| 152 | nd | H | PhCONH | H | PhCH₂ O | H | 578 |
| 153 | nd | H | 4-pyridyl-CONH | H | PhCH₂ O | H | 579 |
| 154 | nd | H | (CF₃CO)₂N | H | PhCH₂ O | H | 666 |
| 155 | nd | HO₂CCH₂CH₂C O | 4-MeO-PhCONH | PhO | H | H | 694 |
| 156 | nd | HO₂CCH₂CH₂C O | PhCONH | PhO | H | H | 664 |
| 157 | nd | HO₂CCH₂CH₂ O | 2-naphthyl-CONH | PhO | H | H | 714 |
| 158 | nd | HO₂CCH₂CH₂C O | 4-Me-PhSO₂NH | PhO | H | H | 714 |
| 159 | nd | HO₂CCH₂CH₂C O | 4-MeO-PhCONH | 2,3-benzo | | H | 652 |
| 160 | nd | HO₂CCH₂CH₂C O | PhCONH | 2,3-benzo | | H | 622 |
| 161 | nd | HO₂CCH₂CH₂C O | 2-naphthyl-CONH | 2,3-benzo | | H | 672 |
| 162 | nd | HO₂CCH₂CH₂C O | 4-Me-PhSO₂NH | 2,3-benzo | | H | 672 |
| 163 | nd | HO₂CCH₂CH₂C O | 4-MeO-PhCONH | H | F | H | 620 |
| 164 | nd | HO₂CCH₂CH₂C O | PhCONH | H | F | H | 590 |
| 165 | nd | HO₂CCH₂CH₂C O | 2-naphthyl-CONH | H | F | H | 640 |
| 166 | nd | HO₂CCH₂CH₂C O | 4-Me-PhSO₂NH | H | F | H | 640 |
| 167 | nd | HO₂CCH₂CH₂C O | 4-MeO-PhCONH | PhCH₂O | H | H | 708 |
| 168 | nd | HO₂CCH₂CH₂C O | PhCONH | PhCH₂O | H | H | 678 |
| 169 | nd | HO₂CCH₂CH₂C O | 2-naphthyl-CONH | PhCH₂O | H | H | 728 |
| 170 | nd | HO₂CCH₂CH₂C O | 4-Me-PhSO₂NH | PhCH₂O | H | H | 728 |

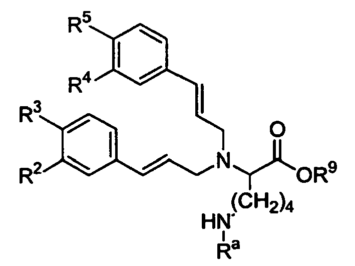

**Table 9**

| cpd | EPO / EBP-Ig %inh @ 50 M | R^{a} | R² | R³ | R⁴ | R⁵ | R⁹ | MS MH + |
|---|---|---|---|---|---|---|---|---|
| 171 | nb | Cbz | H | H | H | H | Me | 527 |
| 172 | 15 | Cbz | H | H | H | H | H | 513 |
| 173 | 5 | Cbz | H | H | H | H | t-Bu | 569 |
| 174 | 23 | Cbz | H | MeO | H | MeO | Me | 587 |
| 175 | 1 | Cbz | | 3,4-benzo | | 3,4-benzo | Me | 627 |
| 176 | -4 | Cbz | PhO | H | PhO | H | Me | 711 |
| 177 | nd | Cbz | 2,3-benzo | | 2,3-benzo | | Me | 627 |
| 178 | 36 | Boc | H | NO₂ | H | NO₂ | Me | 583 |
| 179 | 30 | Boc | H | NO₂ | H | NO₂ | H | 569 |
| 180 | -4 | Boc | PhO | H | PhO | H | Me | 677 |
| 181 | -9 | Boc | 4-t-Bu-PhO | H | 4-t-Bu-PhO | H | Me | 790 |
| 182 | 18 | H | 4-t-Bu-PhO | H | 4-t-Bu-PhO | H | Me | 689 |
| 183 | 36 | Boc | NO₂ | H | NO₂ | H | Me | 583 |
| 184 | 53 | H | NO₂ | H | NO₂ | H | Me | 483 |
| 185 | 29 | H | NH₂ | H | NH₂ | H | Me | 423 |
| 186 | nd | H | n-Bu-CONH | H | n-Bu-CONH | H | Me | 591 |
| 187 | nd | H | 2-furyl-CONH | H | 2-furyl-CONH | H | Me | 611 |
| 188 | nd | H | PhCONH | H | PhCONH | H | Me | 631 |
| 189 | nd | H | 4-Me-PhCONH | H | 4-Me-PhCONH | H | Me | 659 |
| 190 | nd | H | 4-NO₂-PhCONH | H | 4-NO₂-PhCONH | H | Me | 721 |
| 191 | nd | H | 4-Me-PhSO₂NH | H | 4-Me-PhSO₂NH | H | Me | 731 |
| 192 | nd | H | Cbz-NH | H | Cbz-NH | H | Me | 691 |
| 193 | nd | H | 4-Br-PhCONH | H | 4-Br-PhCO | H | Me | 789 |
| 194 | nd | H | 2-MeO-PhCONH | H | 2-MeO-PhCONH | H | Me | 691 |
| 195 | nd | H | 3-MeO-PhCONH | H | 3-MeO-PhCONH | H | Me | 691 |
| 196 | nd | H | 4-MeO-PhCONH | H | 4-MeO-PhCONH | H | Me | 691 |
| 197 | nd | H | CH₃CH=CHCON H | H | CH₃CH=CHCON H | H | Me | 559 |
| 198 | nd | H | C₂F₅CONH | H | C₂F₅CONH | H | Me | 715 |
| 199 | nd | H | 2-naphthyl-CONH H | | 2-naphthyl-CONH H | | Me | 731 |
| 200 | nd | H | EtO₂CCH₂CH₂CO NH | H | EtO₂CCH₂CH₂CO NH | H | Me | 679 |
| 201 | nd | H | CF₃CONH | H | CF₃CONH | H | Me | 615 |
| 202 | nd | H | MeSO₂NH | H | MeSO₂NH | H | Me | 579 |

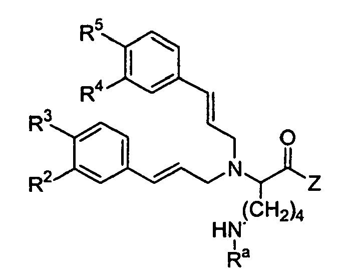

**Table 10**

| cpd | EPO / EBPIg %inh @ 50 M | R^{a} | R² | R³ | R⁴ | R⁵ | Z | MS MH + |
|---|---|---|---|---|---|---|---|---|
| 203 | 37 | Boc | H | H | H | H | 4-(MeCOCH₂CH₂)-PhNH | 640 |
| 204 | -6 | H | H | H | H | H | 4-(MeCOCH₂CH₂)-PhNH | 540 |
| 205 | 26 | H | H | H | H | H | n-Bu-NH | 434 |
| 206 | 17 | 2-MeO-PhCO | H | H | H | H | n-Bu-NH | 568 |
| 207 | 20 | 4-MeO-PhCO | H | H | H | H | n-Bu-NH | 568 |
| 208 | 22 | PhCO | H | H | H | H | n-Bu-NH | 538 |
| 209 | 25 | 2-MeO-PhCO | H | H | H | H | n-Bu-NH | 568 |
| 210 | nd | Boc | H | H | H | H | 4-MeO-PhCH₂CH₂NH | 612 |
| 211 | 62 | H | H | H | H | H | 4-MeO-PhCH₂CH₂NH | 512 |
| 212 | -10 | H | H | H | H | H | n-Pr-NH | 420 |
| 214 | nd | Boc | H | H | H | H | 3,4-di-MeO-PhCH₂CH₂NH | 642 |
| 215 | nd | Boc | H | H | H | H | 3-MeO-PhCH₂CH₂NH | 612 |
| 216 | 10 | Boc | H | H | H | H | 4-(PhCH=CHCH₂O)-PhCH₂NH | 700 |
| 217 | nd | Boc | H | H | H | H | 4-HO-PhCH₂NH | 584 |
| 218 | nd | Boc | H | H | H | H | EtNH | 506 |
| 219 | nd | Boc | H | H | H | H | MeNH | 492 |
| 220 | 45 | H | H | H | H | H | 4-(PhCH=CHCH₂O)-PhCH₂NH | 600 |
| 221 | 48 | H | H | H | H | H | 3,4-di-MeO-PhCH₂CH₂NH | 542 |
| 222 | 56 | H | H | H | H | H | 3-MeO-PhCH₂CH₂NH | 512 |
| 223 | nd | Boc | H | H | H | H | 2-MeO-PhCH₂CH₂NH | 612 |
| 224 | 51 | H | H | H | H | H | 2-MeO-PhCH₂CH₂NH | 512 |
| 225 | 10 | Boc | PhO | H | PhO | H | 4-MeO-PhCH₂CH₂NH | 797 |
| 226 | nd | Boc | H | H | H | H | PhCH₂CH₂NH | 582 |
| 227 | 48 | H | H | H | H | H | PhCH₂CH₂NH | 482 |
| 228 | 21 | PhNHCO | PhO | H | PhO | H | 4-MeO-PhCH₂CH₂NH | 816 |
| 229 | 22 | 4-PhO-PhNHCO | H | H | H | H | 4-MeO-PhCH₂CH₂NH | 723 |
| 230 | 42 | 3,4-di-Cl-PhNHCO | H | H | H | H | 4-MeO-PhCH₂CH₂NH | 700 |
| 231 | 36 | 4-EtO2C-PhNHCO | H | H | H | H | 4-MeO-PhCH₂CH₂NH | 703 |
| 232 | 14 | 4-PhO-PhNHCO | PhO | H | PhO | H | 4-MeO-PhCH₂CH₂NH | 908 |
| 233 | 18 | H | H | NO 2 | H | NO 2 | 3-MeO-PhCH₂CH₂NH | 602 |
| 234 | nd | Boc | H | H | H | H | PhCH₂NH | 568 |
| 235 | 49 | H | H | H | H | H | PhCH₂NH | 468 |
| 236 | nd | Boc | H | Ph | H | Ph | 4-MeO-PhCH₂CH₂NH | 765 |
| 237 | 55 | HO₂CCH₂CH₂CO | H | H | H | H | 3-MeO-PhCH₂CH₂NH | 612 |
| 238 | 39 | H | H | Ph | H | Ph | 4-MeO-PhCH₂CH₂NH | 664 |
| 239 | 46 | H | PhO | H | PhO | H | PhCH₂CH₂NH | 666 |
| 240 | nd | HO₂CCH₂CH₂CH ₂CO | PhO | H | PhO | H | PhCH₂CH₂NH | 780 |
| 285 | 40 | H | H | H | H | H | 4-(NH₂CO)piperidin-1-yl | 489 |

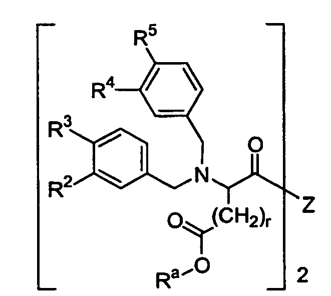

**Table 11**

| cpd | EPO / EBPIg %inh @ 50 M | R^{a} | R² | R³ | R⁴ | R⁵ | Z | r | MS MH₂/2 ]+ |
|---|---|---|---|---|---|---|---|---|---|
| 241 | 2 | t-Bu | H | PhCH₂ O | H | PhCH₂ O | NH(CH₂)₃O(CH₂)₄O(CH₂)₃ NH | 1 | 666 |
| 242 | 1 | t-Bu | H | PhCH₂ O | H | PhCH₂ O | NH(CH₂)₃O(CH₂CH₂O)₂(C H₂)₃NH | 1 | 674 |
| 243 | 75 | H | H | PhCH₂ O | H | PhCH₂ O | NH(CH₂)₃O(CH₂)₄O(CH₂)₃ NH | 1 | 610 |
| 244 | 66 | H | H | PhCH₂ O | H | PhCH₂ O | NH(CH₂)₃O(CH₂CH₂O)₂(C H₂)₃NH | 1 | 618 |
| 245 | 0 | t-Bu | H | PhCH₂ O | H | PhCH₂ O | NH(CH₂)₂O(CH₂)₂O(CH₂)₂ NH | 2 | 652 |
| 246 | 79 | H | H | PhCH₂ O | H | PhCH₂ O | NH(CH₂)₂O(CH₂)₂O(CH₂)₂ NH | 2 | 596 |
| 247 | 47 | H | n-Bu-CONH | H | Ph O | H | NH(CH₂)₂O(CH₂)₂O(CH₂)₂ NH | 2 | 575 |
| 248 | 56 | H | 2-furyl-CONH | H | Ph O | H | NH(CH₂)₂O(CH₂)₂O(CH₂)₂ NH | 2 | 585 |
| 249 | 72 | H | 4-Me-PhCON H | H | Ph O | H | NH(CH₂)₂O(CH₂)₂O(CH₂)₂ NH | 2 | 609 |
| 250 | 78 | H | 4-Me-PhSO₂N H | H | Ph O | H | NH(CH₂)₂O(CH₂)₂O(CH₂)₂ NH | 2 | 645 |

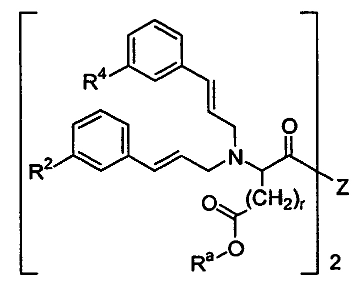

**Table 12**

| cpd | EPO / EBP-Ig %inh @ 50 M | R^{a} | R² | R⁴ | Z | r | MS [MH₂/2 ]+ |
|---|---|---|---|---|---|---|---|
| 251 | 49 | H | H | H | NH(CH₂)₂O(CH₂)₂O(CH₂)₂ NH | 2 | 436 |
| 252 | -4 | t-Bu | 4-t-Bu-PhO | 4-t-Bu-PhO | NH(CH₂)₃O(CH₂)₄O(CH₂)₃ NH | 1 | 803 |
| 253 | -5 | t-Bu | 4-t-Bu-PhO | 4-t-Bu-PhO | NH(CH₂)₃O(CH₂CH₂O)₂(C H₂)₃NH | 1 | 811 |
| 254 | -9 | t-Bu | 4-t-Bu-PhO | 4-t-Bu-PhO | NH(CH₂)₁₀NH | 1 | 787 |
| 255 | 0 | t-Bu | 4-t-Bu-PhO | 4-t-Bu-PhO | NH(CH₂)₁₂NH | 1 | 801 |
| 256 | 10 | t-Bu | 4-t-Bu-PhO | 4-t-Bu-PhO | NH(CH₂)₂O(CH₂)₂O(CH₂)₂ NH | 1 | 789 |

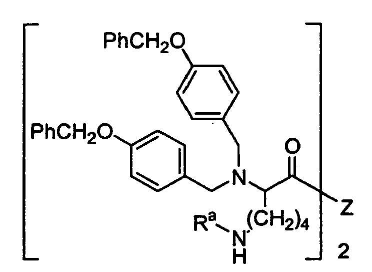

**Table 13**

| cpd | EPO / EBP-Ig %inh @ 50 M | R^{a} | Z | MS [MH₂/2] + |
|---|---|---|---|---|
| 257 | -26 | Boc | NH(CH₂)₃O(CH₂CH₂O)₂(CH₂ )₃NH | 731 |
| 258 | -24 | Boc | NH(CH₂)₃O(CH₂)₄O(CH₂)₃N H | 723 |
| 259 | -13 | Boc | NH(CH₂)₁₂NH | 721 |
| 260 | -12 | Boc | NH(CH₂)₂O(CH₂)₂O(CH₂)₂N H | 695 |
| 261 | 51 | H | NH(CH₂)₂O(CH₂)₂O(CH₂)₂N H | 595 |
| 262 | 93 | HO₂CCH₂CH₂CO | NH(CH₂)₂O(CH₂)₂O(CH₂)₂N H | 695 |
| 263 | 88 | HO₂C(CH₂)₃CO | NH(CH₂)₂O(CH₂)₂O(CH₂)₂N H | 709 |
| 264 | 89 | HO₂CCH₂CMe₂CH₂ CO | NH(CH₂)₂O(CH₂)₂O(CH₂)₂N H | 737 |
| 265 | 65 | HO₂CCH₂CH₂CO | NH(CH₂)₃O(CH₂)₄O(CH₂)₃N H | 723 |
| 266 | 82 | HO₂C(CH₂)₃CO | NH(CH₂)₃O(CH₂)₄O(CH₂)₃N H | 737 |
| 267 | 83 | HO₂CCH₂CMe₂CH₂ CO | NH(CH₂)₃O(CH₂)₄O(CH₂)₃N H | 765 |
| 268 | 40 | HO₂CCH₂CMe2CH₂ CO | NH(CH₂)₁₂NH | 764 |
| 269 | 55 | HO₂CCH₂CH₂CH₂C O | NH(CH₂)₁₂NH | 735 |
| 270 | 56 | HO₂CCH₂CH₂CO | NH(CH₂)₁₂NH | 721 |
| 271 | 77 | HO₂CCH₂CH₂CO | NH(CH₂)₃O(CH₂CH₂O)₂(CH₂ )₃NH | 731 |
| 272 | 78 | HO₂CCH₂CH₂CH₂C O | NH(CH₂)₃O(CH₂CH₂O)₂(CH₂ )₃NH | 745 |

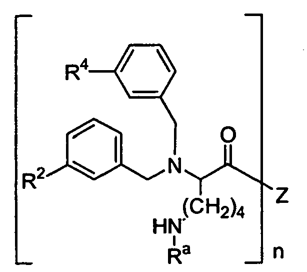

**Table 14**

| cpd | EPO / EBP-Ig %inh @ 50 M | R^{a} | R² | R⁴ | Z | n | MS [MH₂/2 ]+ |
|---|---|---|---|---|---|---|---|
| 273 | nd | HO₂CCH₂CH₂ CO | 4-Me-PhO | 4-Me-PhO | NH(CH₂)₂O(CH₂)₂O(C H₂)₂NH | 2 | 695 |
| 274 | nd | HO₂CCH₂CH₂ CO | PhO | PhO | NH(CH₂)₂O(CH₂)₂O(C H₂)₂NH | 2 | 667 |
| 275 | nd | HO₂CCH₂CH₂ CO | 4-MeO-PhO | 4-MeO-PhO | NH(CH₂)₂O(CH H₂)₂NH | 2 | 727 |
| 276 | nd | HO₂CCH₂CH₂ CO | 4-t-Bu-PhO | 4-t-Bu-PhO | NH(CH₂)₂O(CH₂)₂O(C H₂)₂NH | 2 | 780 |
| 277 | nd | H | PhO | PhO | (NHCH₂CH₂)₃N | 3 | 813 |
| 278 | nd | H | 4-Me-PhO | 4-Me-PhO | (NHCH₂CH₂)₃N | 3 | 855 |
| 279 | nd | H | 4-MeO-PhO | 4-MeO-PhO | (NHCH₂CH₂)₃N | 3 | 903 |
| 280 | nd | HO₂CCH₂CH₂ CO | 4-MeO-PhO | 4-MeO-PhO | (NHCH₂CH₂)₃N | 3 | 1053 |
| 281 | nd | HO₂CCH₂CH₂ CO | 4-Me-PhO | 4-Me-PhO | (NHCH₂CH₂)₃N | 3 | 1005 |
| 282 | nd | HO₂CCH₂CH₂ CO | PhO | PhO | (NHCH₂CH₂)₃N | 3 | 963 |
| 283 | nd | Boc | PhO | PhO | NH(CH₂)₃NMe (CH₂)₃NH | 2 | 666 |
| 284 | nd | Boc | 4-Me-PhO | 4-Me-PhO | NH(CH₂)₃NMe (CH₂)₃NH | 2 | 694 |

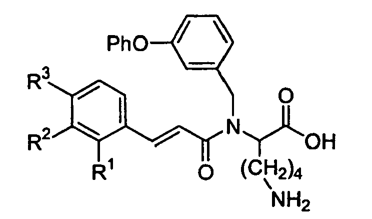

**Table 14**

| cpd | EPO / EBP-Ig %inh @ 50 M | R¹ | R² | R³ | MS, MH+ |
|---|---|---|---|---|---|
| | | | | | |
| 285 | -28 | Me | H | H | 473 |
| 286 | 46 | H | PhCH₂O | H | 565 |
| 287 | 36 | H | 4-MePhO | H | 565 |
| 288 | 27 | H | 4-tBuPhO | H | 607 |
| 289 | 20 | H | H | PhO | 551 |

## Claims

1. A compound of Formula I, for use as a medicament, 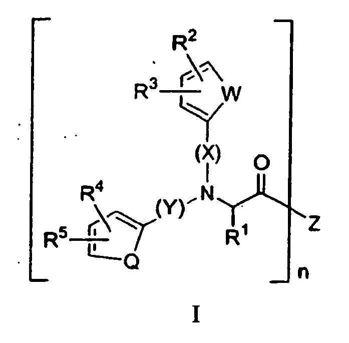 wherein:
R¹
is the side chain of a natural or unnatural α-amino acid where, if said side chain contains a protectable group, that group may be protected with a succinyl, glutaryl, 3,3-dimethylglutaryl, C₁₋₅alkyl, C₁₋₅alkoxycarbonyl, acetyl, N-(9-fluorenylmethoxycarbonyl), trifluoroacetyl, omega-carboxyC₁₋₅ alkylcarbonyl, t-butoxycarbonyl, benzyl, benzyloxycarbonyl, 2-chlorobenzyloxycarbonyl, phenylsulfonyl, ureido, t-butyl, cinnamoyl, trityl, 4-methyltrityl, 1-(4,4-dimethyl-2,6-dioxo-cyclohexylidene)ethyl, tosyl, 4-methoxy-2,3,6-trimethylbenzenesulfonyl, phenylureido or substituted phenylureido (where the phenyl substituents are selected from phenoxy, halo and C₁₋₅alkoxycarbonyl) group;
R2 and R3; and R⁴ and R⁵; independently
may be taken together to form a six-membered aromatic ring which is fused to the depicted ring, or
are independently selected from:
hydrogen, C₁₋₅alkyl, C₁₋₅alkoxy, hydroxy, halo, trifluoromethyl, nitro, amino, phenyl, phenoxy, phenylC₁₋₅alkyl, phenylC₁₋₅alkoxy,
substituted phenyl substituted phenoxy, substituted phenylC₁₋₅ alkyl or substituted phenylC₁₋₅alkoxy (where the substituents are selected from C₁₋₅alkyl, C₁₋₅alkoxy, hydroxy, halo, trifluoromethyl, nitro, nitrile and amino) and
substituted amino (where the substituents are selected from one or more of C₁₋₅alkyl, halosubstitutedC₁₋₅alkyl, C₁₋₅alkynyl, C₁₋₅alkenyl, phenyl, phenylC₁₋₅alkyl, C₁₋₅alkylcarbonyl, halo substituted C₁₋₅alkylcarbonyl, carboxyC₁₋₅alkyl, C₁₋₅alkoxyC₁₋₅alkyl, cinnamoyl, naphthylcarbonyl, furylcarbonyl, pyridylcarbonyl, C₁₋₅alkylsulfonyl, phenylcarbonyl, phenylC₁₋₅alkylcarbonyl, phenylsulfonyl, phenylC₁₋₅ alkylsulfonyl, substituted phenylcarbonyl, substituted phenylC₁₋₅ alkylcarbonyl, substituted phenylsulfonyl, substituted phenylC₁₋₅ alkylsulfonyl, substituted phenyl and substituted phenylC₁₋₅alkyl [where the aromatic phenyl, phenylC₁₋₅alkyl, phenylcarbonyl, phenylC₁₋₅ alkylcarbonyl, phenylsulfonyl and phenylC₁₋₅alkylsulfonyl substitutents are independently selected from one to five members of C₁₋₅alkyl, C₁₋₅ alkoxy, hydroxy, halogen, trifluoromethyl, nitro, nitrile and amino]);
W and Q
are independently -CH=CH-, -S- or -CH=N-;
X and Y
are independently carbonyl, C₁₋₅alkyl, C₁₋₅alkenyl, C₁₋₅alkenylcarbonyl or (CH₂)ₘ-C(O)- where m is 2-5;
n
is 1,2, or 3;
Z
is hydroxy, C₁₋₅alkoxy, phenoxy, phenylC₁₋₅alkoxy, amino, C₁₋₅ alkylamino, diC₁₋₅alkylamino, phenylamino, phenylC₁₋₅alkylamino, pipehdin-1-yl, substituted piperidin-1-yl (where the substituents are selected from C₁₋₅alkyl C₁₋₅alkoxy, halo, aminocarbonyl, C₁₋₅alkoxycarbonyl and oxo), substituted phenylC₁₋₅alkylamino (where the aromatic substitutents are selected from C₁₋₅ alkyl, C₁₋₅alkoxy, phenylC₁₋₅alkenyloxy, hydroxy, halogen, trifluoromethyl, nitro, nitrile and amino), substituted phenoxy (where the aromatic substitutents are selected from C₁₋₅alkyl, C₁₋₅alkoxy, hydroxy, halogen, trifluoromethyl, nitro, nitrile, and amino), substituted phenylC₁₋₅alkoxy (where the aromatic substitutents are selected from C₁₋₅alkyl, C₁₋₅alkoxy hydroxy, halogen, trifluoromethyl, nitro, nitrile and amino), -OCH₂CH₂(OCH₂CH₂)OCH₂CH₂O-, -NHCH₂CH₂(OCH₂CH₂)ₛOCH₂CH₂NH-, -NH(CH₂)ₚO(CH₂)_{q}O(CH₂)ₚNH-, -NH(CH₂)_{q}NCH₃(CH₂)ₛNH-, -NH(CH₂)ₛNH- or -(NH(CH₂)ₛ)₃N, where s, p, and q are independently selected from 1-7
with the proviso that if n is 2, Z is not hydroxy, C₁₋₅alkoxy, amino, C₁₋₅alkylamino, diC₁₋₅alkylamino, phenylamino, phenylC₁₋₅alkylamino or piperidin-1-yl
with the further proviso that if n is 3, Z is (NH(CH₂)ₛ)₃N.
and salts thereof.

2. A compound for use as a medicament according to claim 1, wherein R¹ is the side chain of lysine, ornithine, arginine, aspartic acid, glutamic acid, glutamine, cysteine, methionine, serine or threonine.

3. A compound for use as a medicament according to claim 1, wherein R² and R³ are independently phenoxy, substituted phenoxy, benzyloxy or substituted benzyloxy.

4. A compound for use as a medicament according to claim 1, wherein R⁴ and R⁵ are independently phenoxy, substituted phenoxy, benzyloxy or substituted benzyloxy.

5. A compound for use as a medicament according to claim 1, wherein W is -CH=CH-.

6. A compound for use as a medicament according to claim 1, wherein Q is -CH=CH-.

7. A compound for use as a medicament according to claim 1, wherein X is C₁₋₅ alkenyl or -CH₂-.

8. A compound for use as a medicament according to claim 1, wherein Y is C₁₋₅ alkenyl or -CH₂-.

9. A compound for use as a medicament according to claim 1, wherein n is 1 or 2.

10. A compound for use as a medicament according to claim 1, wherein Z is hydroxy, methoxy, phenethylamino, substituted phenethylamino or -NH(CH₂)₂O(OH₂)₂O(CH₂)₂NH-.

11. A compound for use as a medicament according to claim 1, which is 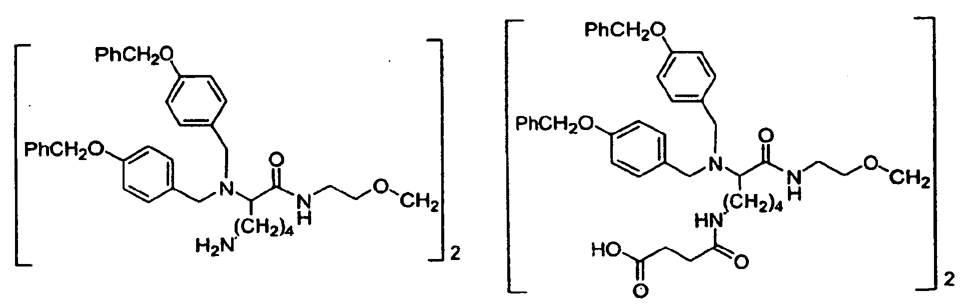 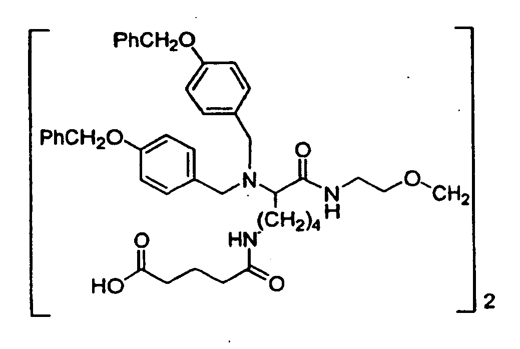 and salts thereof.

12. A pharmaceutical composition comprising a compound of Formula I as defined in any one of claims 1 to 11 and a pharmaceutically acceptable carrier.

13. A method for making a pharmaceutical composition which comprises mixing a compound of Formula I as defined in any one of claims 1 to 11 and a pharmaceutically acceptable carrier.

14. An *in vitro* method for modulating an EPO receptor comprising contacting the EPO receptor with an EPO receptor-modulating amount of a compound of Formula I as defined in any one of claims 1 to 11.

15. Use of a compound of Formula I as defined in any one of claims 1 to 11 in the manufacture of a medicament for the treatment of a disease or condition mediated by the EPO receptor.

16. A compound of Formula I as defined in any one of claims 1 to 11, provided that the compound is not: 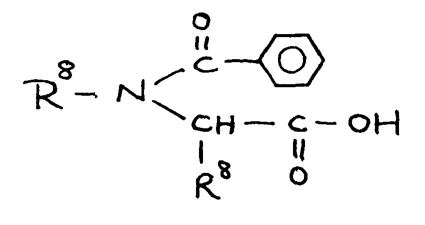 wherein R⁸ is benzyl, 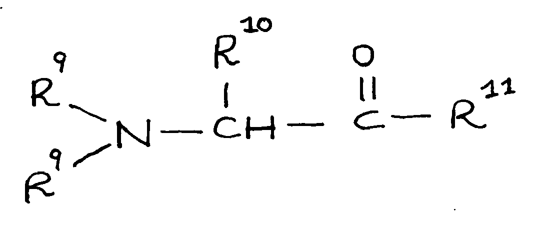 wherein R⁹ is benzyl, R¹⁰ is isobutyl, benzyl, phenethyl, cyclohexylmethyl, cyclohexyl, isopropyl, secondary butyl or methyl and R¹¹ is hyroxy or methoxy, or 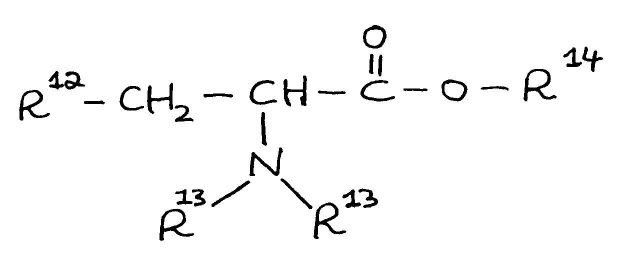 wherein R¹² is 4-methoxy phenyl, R¹³ is benzyl and R¹⁴ is hydrogen or benzyl.

## Patentansprüche

1. Verbindung von Formel I, zur Verwendung als ein Arzneimittel, 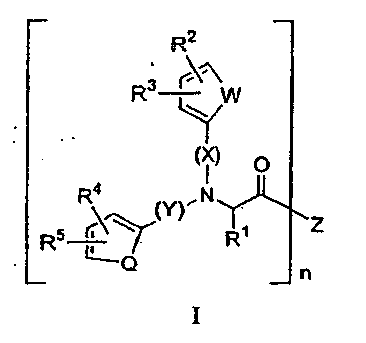 worin:
R¹ die Seitenkette einer natürlichen oder nicht-natürlichen α-Aminosäure ist, wobei, wenn besagte Seitenkette eine schützbare Gruppe enthält, diese Gruppe geschützt sein kann mit einer Succinyl-, Glutaryl-, 3,3-Dimethylglutaryl-, C₁₋₅-Alkyl-, C₁₋₅-Alkoxycarbonyl-, Acetyl-, N-(9-Fluorenylmethoxycarbonyl)-, Trifluoracetyl-, omega-Carboxy-C₁₋₅-alkylcarbonyl-, t-Butoxycarbonyl-, Benzyl-, Benzyloxycarbonyl-, 2-Chlorbenzyloxycarbonyl-, Phenylsulfony-, Ureido-, t-Butyl-, Cinnamoyl-, Trityl-, 4-Methyltrityl, 1-(4,4-Dimethyl-2,6-dioxocyclohexyliden)ethyl-, Tosyl-, 4-Methoxy-2,3,6-trimethylbenzolsulfonyl-, Phenylureido- oder substituierten Phenylureidogruppe (wobei die Phenyl-Substituenten ausgewählt sind aus Phenoxy, Halo und C₁₋₅-Alkoxycarbonyl);
R² und R³; und R⁴ und R⁵; unabhängig
zusammengenommen sein können, um einen sechsgliedrigen aromatischen Ring zu bilden, der an den dargestellten Ring kondensiert ist, oder
unabhängig ausgewählt sind aus:
Wasserstoff, C₁₋₅-Alkyl, C₁₋₅-Alkoxy, Hydroxy, Halo, Trifluormethyl, Nitro, Amino, Phenyl, Phenoxy, Phenyl-C₁₋₅-alkyl, Phenyl-C₁₋₅-akoxy,
substituiertem Phenyl, substituiertem Phenoxy, substituiertem Phenyl-C₁₋₅-alkyl oder substituiertem Phenyl-C₁₋₅-alkoxy (wobei die Substituenten ausgewählt sind aus C₁₋₅-Alkyl, C₁₋₅-Alkoxy, Hydroxy, Halo, Trifluormethyl, Nitro, Nitril und Amino) und
substituiertem Amino (wobei die Substituenten ausgewählt sind aus einem oder mehreren von C₁₋₅-Alkyl, halosubstituiertem C₁₋₅-Alkyl, C₁₋₅-Alkinyl, C₁₋₅-Alkenyl, Phenyl, Phenyl-C₁₋₅-alkyl, C₁₋₅-Alkylcarbonyl, halosubstituiertem C₁₋₅-Alkylcarbonyl, Carboxy-C₁₋₅-alkyl, C₁₋₅-Alkoxy-C₁₋₅-alkyl, Cinnamoyl, Naphthylcarbonyl, Furylcarbonyl, Pyridylcarbonyl, C₁₋₅-Alkylsulfonyl, Phenylcarbonyl, Phenyl-C₁₋₅-alkylcarbonyl, Phenylsulfonyl, Phenyl-C₁₋₅-alkylsulfonyl, substituiertem Phenylcarbonyl, substituiertem Phenyl-C₁₋₅-alkylcarbonyl, substituiertem Phenylsulfonyl, substituiertem Phenyl-C₁₋₅-alkylsulfonyl, substituiertem Phenyl und substituiertem Phenyl-C₁₋₅-alkyl [wobei die aromatischen Phenyl-, Phenyl-C₁₋₅-alkyl-, Phenylcarbonyl-, Phenyl-C₁₋₅-alkylcarbonyl-, Phenylsulfonyl- und Phenyl-C₁₋₅-alkylsulfonyl-Substituenten unabhängig ausgewählt sind aus einer bis fünf Gruppen von C₁₋₅-Alkyl, C₁₋₅-Alkoxy, Hydroxy, Halogen, Trifluormethyl, Nitro, Nitril und Amino]);
W und Q unabhängig -CH=CH-, -S- oder -CH=N- sind;
X und Y unabhängig Carbonyl, C₁₋₅-Alkyl, C₁₋₅-Alkenyl, C₁₋₅-Alkenylcarbonyl oder (CH₂)ₘ-C(O)- sind, wobei m 2-5 ist;
n 1, 2 oder 3 ist;
Z Hydroxy, C₁₋₅-Alkoxy, Phenoxy, Phenyl-C₁₋₅-alkoxy, Amino, C₁₋₅-Alkylamino, Di-C₁₋₅-alkylamino, Phenylamino, Phenyl-C₁₋₅-alkylamino, Piperidin-1-yl, substituiertes Piperidin-1-yl (wobei die Substituenten ausgewählt sind aus C₁₋₅-Alkyl, C₁₋₅-Alkoxy, Halo, Aminocarbonyl, C₁₋₅-Alkoxycarbonyl und Oxo), substituiertes Phenyl-C₁₋₅-alkylamino (wobei die aromatischen Substituenten ausgewählt sind aus C₁₋₅-Alkyl, C₁₋₅-Alkoxy, Phenyl-C₁₋₅-alkenyloxy, Hydroxy, Halogen, Trifluormethyl, Nitro, Nitril und Amino), substituiertes Phenoxy (wobei die aromatischen Substituenten ausgewählt sind aus C₁₋₅-Alkyl, C₁₋₅-Alkoxy, Hydroxy, Halogen, Trifluormethyl, Nitro, Nitril und Amino), substituiertes Phenyl-C₁₋₅-alkoxy (wobei die aromatischen Substituenten ausgewählt sind aus C₁₋₅-Alkyl, C₁₋₅-Alkoxy, Hydroxy, Halogen, Trifluormethyl, Nitro, Nitril und Amino), -OCH₂CH₂(OCH₂CH₂)OCH₂CH₂O-, -NHCH₂CH₂(OCH₂CH₂)ₛOCH₂CH₂NH-, -NH(CH₂)ₚO(CH₂)_{q}O(CH₂)pNH-, -NH(CH₂)_{q}NCH₃(CH₂)ₛNH-, -NH(CH₂)ₛNH- oder -(NH(CH₂)ₛ)₃N, worin s, p und q unabhängig ausgewählt sind aus 1-7,
mit der Maßgabe, daß, wenn n 2 ist, Z nicht Hydroxy, C₁₋₅-Alkoxy, Amino, C₁₋₅-Alkylamino, Di-C₁₋₅-alkylamino, Phenylamino, Phenyl-C₁₋₅-alkylamino oder Piperidin-1-yl ist,
mit der weiteren Maßgabe, daß, wenn n 3 ist, Z (NH(CH₂)ₛ)₃N ist,
und Salze derselben.

2. Verbindung zur Verwendung als ein Arzneimittel nach Anspruch 1, **dadurch gekennzeichnet, daß** R¹ die Seitenkette von Lysin, Ornithin, Arginin, Asparaginsäure, Glutaminsäure, Glutamin, Cystein, Methionin, Serin oder Threonin ist.

3. Verbindung zur Verwendung als ein Arzneimittel nach Anspruch 1, **dadurch gekennzeichnet, daß** R² und R³ unabhängig Phenoxy, substituiertes Phenoxy, Benzyloxy oder substituiertes Benzyloxy sind.

4. Verbindung zur Verwendung als ein Arzneimittel nach Anspruch 1, **dadurch gekennzeichnet, daß** R⁴ und R⁵ unabhängig Phenoxy, substituiertes Phenoxy, Benzyloxy oder substituiertes Benzyloxy sind.

5. Verbindung zur Verwendung als ein Arzneimittel nach Anspruch 1, **dadurch gekennzeichnet, daß** W -CH=CH- ist.

6. Verbindung zur Verwendung als ein Arzneimittel nach Anspruch 1, **dadurch gekennzeichnet, daß** Q -CH=CH- ist.

7. Verbindung zur Verwendung als ein Arzneimittel nach Anspruch 1, **dadurch gekennzeichnet, daß** X C₁₋₅-Alkenyl oder -CH₂- ist.

8. Verbindung zur Verwendung als ein Arzneimittel nach Anspruch 1, **dadurch gekennzeichnet, daß** Y C₁₋₅-Alkenyl oder -CH₂- ist.

9. Verbindung zur Verwendung als ein Arzneimittel nach Anspruch 1, **dadurch gekennzeichnet, daß** das n 1 oder 2 ist.

10. Verbindung zur Verwendung als ein Arzneimittel nach Anspruch 1, **dadurch gekennzeichnet, daß** Z Hydroxy, Methoxy, Phenethylamino, substituiertes Phenethylamino oder -NH(CH₂)₂O(OH₂)₂O(CH₂)₂NH- ist.

11. Verbindung zur Verwendung als ein Arzneimittel nach Anspruch 1, die 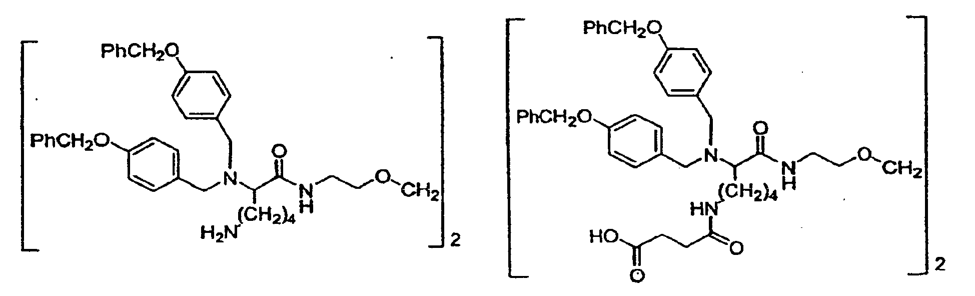 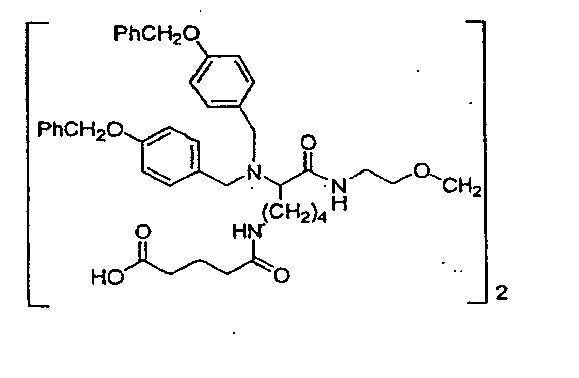 ist, und Salze derselben.

12. Pharmazeutische Zusammensetzung, die eine Verbindung von Formel I, wie definiert in einem der Ansprüche 1 bis 11, und einen pharmazeutisch annehmbaren Trägerstoff umfaßt.

13. Verfahren zur Herstellung einer pharmazeutischen Zusammensetzung, welches das Mischen einer Verbindung von Formel I, wie definiert in einem der Ansprüche 1 bis 11, und eines pharmazeutisch annehmbaren Trägerstoffes umfaßt.

14. In-vitro-Verfahren zur Modulierung eines EPO-Rezeptors, welches das Inkontaktbringen des EPO-Rezeptors mit einer den EPO-Rezeptor modulierenden Menge einer Verbindung von Formel I, wie definiert in einem der Ansprüche 1 bis 11, umfaßt.

15. Verwendung einer Verbindung von Formel I, wie definiert in einem der Ansprüche 1 bis 11, bei der Herstellung eines Arzneimittels zur Behandlung einer Erkrankung oder eines Zustandes, die/der durch den EPO-Rezeptor vermittelt wird.

16. Verbindung nach Formel I, wie definiert in einem der Ansprüche 1 bis 11, vorausgesetzt, daß die Verbindung nicht ist: 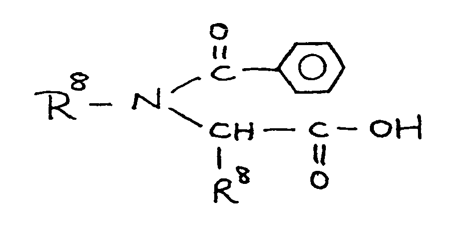 worin R⁸ Benzyl 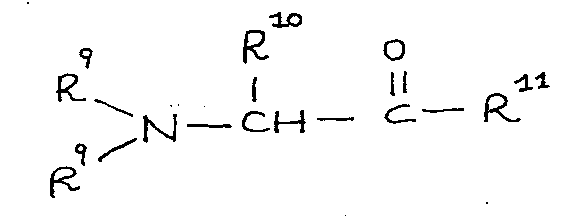 worin R⁹ Benzyl ist, R¹⁰ Isobutyl, Benzyl, Phenethyl, Cyclohexylmethyl, Cyclohexyl, Isopropyl, sekundäres Butyl oder Methyl ist und R¹¹ Hydroxy oder Methoxy ist, oder 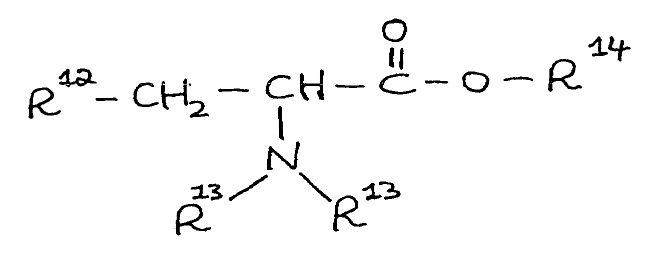 ist, worin R¹² 4-Methoxyphenyl ist, R¹³ Benzyl ist und R¹⁴ Wasserstoff oder Benzyl ist.

## Revendications

1. Composé de la Formule I, à utiliser comme médicament, 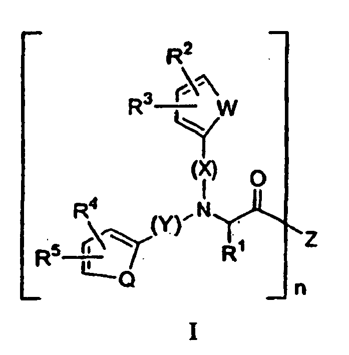 où:
R¹ est la chaîne latérale d'un acide α-aminé naturel ou non naturel où, si ladite chaîne naturelle contient un groupe pouvant être protégé, ce groupe peut être protégé par un groupe succinyle, gluratyle, 3,3-diméthylglutaryle, alkyleC₁₋₅, alcoxycarbonyle C₁₋₅, acétyle, N-(9-fluorénylméthoxycarbonyle), trifluoroacétyle, oméga-carboxy alkylcarbonyleC₁₋₅, *t*-butoxycarbonyle, benzyle, benzyloxycarbonyle, 2-chlorobenzyloxycarbonyle, phénylsulfonyle, uréido, *t*-butyle, cinnamoyle, trityle, 4-méthyltrityle, 1-(4,4-diméthyl-2,6-dioxo-cyclohexylidène)éthyle, tosyle, 4-méthoxy-2,3,6-triméthylbenzènesulfonyle, phényluréido ou phényluréido substitué (où les substituants phényles sont sélectionnés parmi phénoxy, halo et alcoxycarbonyle C₁₋₅);
R2 et R3; et R⁴ et R⁵; indépendamment
peuvent être pris ensemble pour former un cycle aromatique à six membres qui est fusionné au cycle représenté, ou
sont indépendamment sélectionnés parmi:
hydrogène, alkyleC₁₋₅, alcoxyC₁₋₅, hydroxy, halo, trifluorométhyle, nitro, amino, phényle, phénoxy, phénylalkyleC₁₋₅, phénylalcoxyC₁₋₅,
phényle substitué phénoxy substitué, phénylalkyleC₁₋₅ substitué ou phénylalcoxyC₁₋₅ substitué (où les substituants sont sélectionnés parmi alkyleC₁₋₅, alcoxyC₁₋₅, hydroxy, halo, trifluorométhyle, nitro, nitrile et amino) et
amino substitué (où les substituants sont sélectionnés parmi un ou plusieurs de alkyleC₁₋₅, alkyleC₁₋₅ halosubstitué, alkynyleC₁₋₅, alcényleC₁₋₅, phényle, phénylalkyleC₁₋₅, alkylcarbonyleC₁₋₅, halo alkylcarbonyle C₁₋₅ substitué, carboxyalkyle C₁₋₅, alcoxyC₁₋₅alkyleC₁₋₅, cinnamoyle, naphtylcarbonyle, furylcarbonyle, pyridylcarbonyle, alkylsulfonyleC₁₋₅, phénylcarbonyle, phénylalkylcarbonyleC₁₋₅, phénylsulfonyle, phénylalkylsulfonyleC₁₋₅, phénylcarbonyle substitué, phénylalkylcarbonyle substitué (C₁₋₅), phénylsulfonyle substitué, phénylalkylsulfonyleC₁₋₅ substitué, phényle substitué et phénylalkyle C₁₋₅ substitué [où le phényl aromatique, le phénylalkyleC₁₋₅, le phénylcarbonyle, le phénylalkylcarbonyleC₁₋₅, le phénylsulfonyle et les phénylalkylsulfonyleC₁₋₅ comme substituants sont indépendamment sélectionnés parmi un à cinq membres d'alkyleC₁₋₅, alcoxyC₁₋₅, hydroxy, halogène, trifluorométhyle, nitro, nitrile et amino]);
W et Q
sont indépendamment -CH=CH-, -S- ou -CH=N-;
X et Y
sont indépendamment carbonyle, alkyleC₁₋₅, alcényle C₁₋₅, alcénylcarbonyleC₁₋₅ ou (CH₂)ₘ-C(O)- où m est 2-5;
n
est 1,2, ou 3;
Z
est hydroxy, alcoxyC₁₋₅, phénoxy, phénylalcoxyC₁₋₅, amino, alkylaminoC₁₋₅, dialkylaminoC₁₋₅, phénylamino, phénylalkylaminoC₁₋₅, piperidin-1-yle, piperidin-1-yle substitué (où les substituants sont sélectionnés parmi alkyleC₁₋₅, alcoxyC₁₋₅, halo, aminocarbonyle, alcoxycarbonyleC₁₋₅ et oxo), phénylalkylaminoC₁₋₅ substitué (où les substituants aromatiques sont sélectionnés parmi alkyleC₁₋₅, alcoxyC₁₋₅, phénylalcényloxyC₁₋₅, hydroxy, halogène, trifluorométhyle, nitro, nitrile et amino), phénoxy substitué (où les substituants aromatiques sont sélectionnés parmi alkyleC₁₋₅, alcoxyC₁₋₅, hydroxy, halogène, trifluorométhyle, nitro, nitrile, et amino), phénylalcoxy C₁₋₅ substitué (où les substituants aromatiques sont sélectionnés parmi alkyleC_{1-5,} alcoxyC₁₋₅, hydroxy, halogène, trifluorométhyle, nitro, nitrile et amino), -OCH₂CH₂(OCH₂CH₂) OCH₂CH₂O-, -NHCH₂CH₂(OCH₂CH₂)OCH₂CH₂NH-, -NH(CH₂)ₚO(CH₂)_{q} O(CH₂)ₚNH-, -NH (CH₂)_{q}NCH₃(CH₂)ₛNH-, -NH (CH₂)ₛ-NH- ou -(NH (CH₂)ₛ)₃N, où s, p, et q sont indépendamment sélectionnés parmi 1-7
à condition que si n est 2, Z ne soit pas hydroxy, alcoxy C₁₋₅, amino, alkylaminoC₁₋₅, dialkylaminoC₁₋₅, phénylamino, phénylalkylaminoC₁₋₅ ou pipéridin-1-yle
à condition de plus que si n est 3, Z soit (NH(CH₂)ₛ)₃N.
et leurs sels.

2. Composé à utiliser comme médicament selon la revendication 1, où R¹ est la chaîne latérale de lysine, ornithine, arginine, acide aspartique, acide glutamique, glutamine, cystéine, méthionine, sérine ou thréonine.

3. Composé à utiliser comme médicament selon la revendication 1, où R₂ et R₃ sont indépendamment phénoxy, phénoxy substitué, benzyloxy ou benzyloxy substitué.

4. Composé à utiliser comme médicament selon la revendication 1, où R⁴ et R⁵ sont indépendamment phénoxy, phénoxy substitué, benzyloxy ou benzyloxy substitué.

5. Composé à utiliser comme médicament selon la revendication 1, où W est -CH=CH-.

6. Composé à utiliser comme médicament selon la revendication 1, où Q est -CH=CH-.

7. Composé à utiliser comme médicament selon la revendication 1, où X est alcényle C₁₋₅ ou -CH₂-.

8. Composé à utiliser comme médicament selon la revendication 1, où Y est alcényle C₁₋₅ ou -CH₂-.

9. Composé à utiliser comme médicament selon la revendication 1, où n est 1 ou 2.

10. Composé à utiliser comme médicament selon la revendication 1, où Z est hydroxy, méthoxy, phénéthylamino, phénéthylamino substitué ou -NH(CH₂)₂O(OCH₂)₂O(CH₂)₂NH-,

11. Composé à utiliser comme médicament selon la revendication 1, qui est 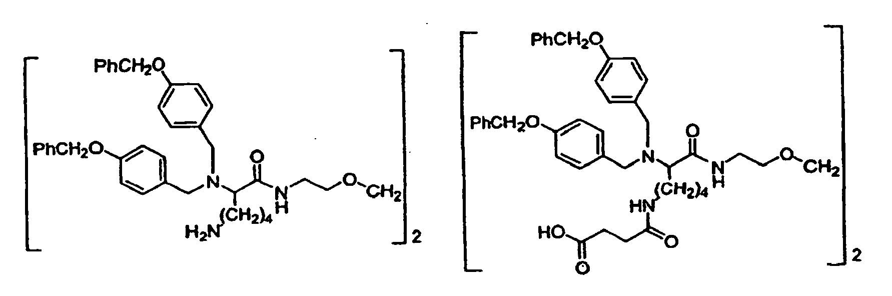 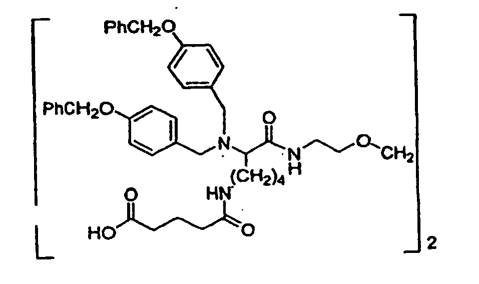 et leurs sels.

12. Composition pharmaceutique comprenant un composé de Formule I tel que défini dans l'une quelconque des revendications 1 à 11 et un support pharmaceutiquement acceptable.

13. Méthode de production d'une composition pharmaceutique qui comprend le mélange d'un composé de Formule I tel que défini dans l'une quelconque des revendications 1 à 11 et d'un support pharmaceutiquement acceptable.

14. Méthode *in vitro* pour moduler un récepteur de EPO comprenant la mise en contact du récepteur de EPO avec une quantité de modulation du récepteur de EPO d'un composé de Formule I tel que défini dans l'une quelconque des revendications 1 à 11.

15. Utilisation d'un composé de Formule I tel que défini dans l'une quelconque des revendications 1 à 11 dans la fabrication d'un médicament pour le traitement d'une maladie ou d'une condition provoquée par le récepteur de EPO.

16. Composé de Formule I tel que défini dans l'une quelconque des revendications 1 à 11, à condition que le composé ne soit pas: 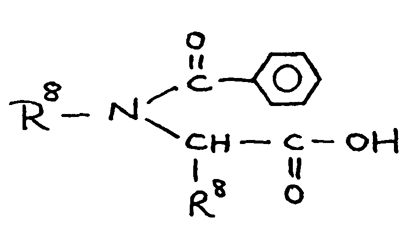 où R⁸ est benzyle, 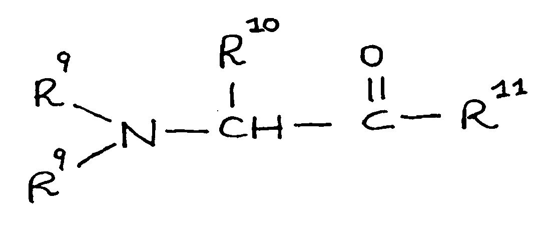 où R⁹ est benzyle, R¹⁰ est isobutyle, benzyle, phénéthyle, cyclohexylméthyle, cyclohexyle, isopropyle, butyle secondaire ou méthyle et R¹¹ est hydroxy ou méthoxy, ou 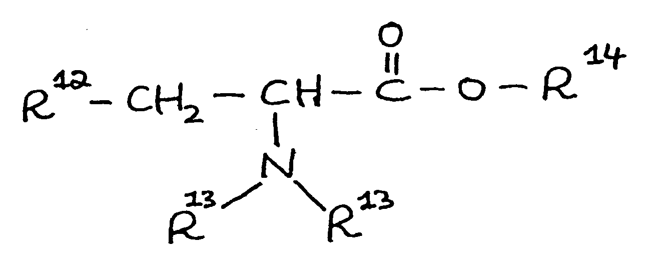 où R¹² est 4-méthoxy phényle, R¹³ est benzyle et R¹⁴ est hydrogène ou benzyle.
